# EUROPEAN PATENT SPECIFICATION

(11) **EP 2 582 793 B1**
(45) Date of publication and mention of the grant of the patent: **06.09.2017**
(21) Application number: 11796365.2
(22) Date of filing: 15.06.2011
(51) Int. Cl.: C12N 5/074, C12N 5/10, C12N 15/65, C12N 15/85, C12Q 1/02, G01N 33/15

(54) **A COMPENDIUM OF READY-BUILT STEM CELL MODELS FOR INTERROGATION OF BIOLOGICAL RESPONSE**
KOMPENDIUM AUS FERTIG GEBAUTEN STAMMZELLMODELLEN ZUR ABFRAGE BIOLOGISCHER REAKTIONEN
COMPENDIUM DE MODÈLES DE CELLULES SOUCHES PRÉFABRIQUÉS POUR L'INTERROGATION D'UNE RÉPONSE BIOLOGIQUE

(30) Priority: 15.06.2010 US 354878 P
(43) Date of publication of application: 24.04.2013
(73) Proprietor: Cellular Dynamics International, Inc., Madison, WI 53711 (US)
(72) Inventor: NUWAYSIR, Emile, Madison, WI 53711 (US); KENDRICK-PARKER, Chris, Madison, WI 53711 (US); SEAY, Nicholas, Madison, WI 53711 (US)
(74) Representative: Vossius & Partner Patentanwälte Rechtsanwälte mbB
(86) International application number: PCT/US2011/040519
(87) International publication number: WO 2011/159797

(56) References cited:
- WO-A2-2004/035774
- SHIRAKI N ET AL: "Guided differentiation of embryonic stem cells into Pdx1-expressing regional-specific definitive endoderm", STEM CELLS, ALPHAMED PRESS, DAYTON, OH, US, vol. 26, no. 4, 1 April 2008 (2008-04-01), pages 874-885, XP002547894, ISSN: 1066-5099, DOI: 10.1634/STEMCELLS.2007-0608 [retrieved on 2008-01-31]
- LACOSTE, A. ET AL.: 'An Efficient and Reversible Transposable System for Ge ne Delivery and Lineage-Specific Differentiation in Human Embryonic Stem Cel Is' CELL STEM CELL vol. 5, 04 September 2009, pages 332 - 342, XP055099142
- XUE, H. ET AL.: 'A Targeted Neuroglial Reporter Line Generated by Homologou s Recombination in Human Embryonic Stem Cells' STEM CELLS vol. 27, 14 May 2009, pages 1836 - 1846, XP055054386
- GERRARD, L. ET AL.: 'Stably Transfected Human Embryonic Stem Cell Clones Ex press OCT4-Specific Green Fluorescent Protein and Maintain Self-Renewal and Pluripotency' STEM CELLS vol. 23, 2005, pages 124 - 133, XP002502212
- ZWATA, T.P. ET AL.: 'Homologous Recombination in Human Embryonic Stem Cells' NATURE BIOTECHNOLOLGY vol. 21, March 2003, pages 319 - 321, XP002427413
- Ebert Et Al.,: "The human stem cells and drug screening: opportunities and challenges", Nature reviews, vol. 9, 1 May 2010 (2010-05-01), pages 1-5, XP055245095,
- Sabrina C. Desbordes ET AL: "High-Throughput Screening Assay for the Identification of Compounds Regulating Self-Renewal and Differentiation in Human Embryonic Stem Cells", Cell Stem Cell, vol. 2, no. 6, 1 June 2008 (2008-06-01), pages 602-612, XP55244850, AMSTERDAM, NL ISSN: 1934-5909, DOI: 10.1016/j.stem.2008.05.010

## Description

### BACKGROUND OF THE INVENTION

### 1. Field of the Invention

The present invention relates generally to the field of molecular biology, stem cells and differentiated cells. More particularly, it concerns engineered stem cell lines that can be used to study biological and/or pharmaceutical response.

### 2. Description of Related Art

A key unmet need in biomedical research and pharmaceutical development is reliably available, cost-effective and predictive models for determining biological response under diverse conditions as well as metabolic and toxicological properties of drug compounds. Current *in vitro* models such as primary cell culture suffer from inconsistent availability and significant phenotypic variability. Current methods used to make cells into cell lines can render the responses of the cells non-authentic. *In vivo* animal models are prohibitively expensive, have low throughput, and are often not predictive for humans.

Previously, high-throughput screening assays for the identification of compounds regulating self-renewal and differentiation in human embryonic stem cells have been described; Desborades et al., Cell Stem Cell, 2, 602-612, 2008.

Therefore, there is a need for production of various cell types in an easy-to-assay format for therapeutic and research use.

### SUMMARY OF THE INVENTION

The present invention overcomes a major deficiency in the art in providing pluripotent stem cells expressing one or more selectable or screenable marker(s) under the control of one or more condition-responsive regulatory elements.
The present invention relates to the embodiment as characterized in the claims. Thus, it relates, *inter alia,* to a method of testing a compound for its effect on differentiation of specific cells or tissue types, comprising the steps of:
(a) providing an *in vitro* set of cell lines comprising at least a first and second pluripotent stem cell line, wherein said first and second lines respectively comprise an integrated exogenous expression cassette, wherein the exogenous expression cassettes from the first and second lines comprise a selectable or screenable marker under the control of a differentiation-responsive regulatory element, wherein the differentiation-responsive regulatory element of said exogenous expression cassette of the first cell line is different from the differentiation-responsive regulatory element of the exogenous expression cassette of the second cell line, such that the marker of the cassette in the first cell line is expressed only if the cell is in a first differentiation state and the marker of the cassette in the second cell line is expressed only if the cell is in a second differentiation state and wherein the expression of the selectable or screenable markers from the exogenous expression cassettes of the first and second cell lines can be screened or selected using the same method,
(b) culturing the pluripotent stem cell lines under a differentiation condition in the presence of a test compound;
(c) determining the expression of the selectable or screenable marker marker for the effect of the testing compound on the differentiation of the pluripotent stem cell to the selected cell lineages or tissue types;
wherein the pluripotent stem cells comprise one or more human induced pluripotent stem (iPS) cells.
Thus, there is disclosed a pluripotent stem cell line comprising a first and second exogenous expression cassette each comprising a selectable or screenable marker under the control of a condition-responsive regulatory element. The condition-responsive regulatory element of said first exogenous expression cassette is different from the condition-responsive regulatory element of said second exogenous expression cassette. For example, the condition-responsive regulatory elements can comprise a differentiation-responsive regulatory element (e.g., a tissue or cell lineage specific promoter) and a drug-responsive regulatory element, such as a drug receptor, drug target, or drug signaling pathway-responsive regulatory element.

There is also disclosed an *in vitro* set of cell lines comprising at least a first and second cell line, in certain aspects, for being able to simultaneously study differentiation, drug response or drug toxicity of various cell types in a large scale. In a particular aspect, the first and second lines each comprise an exogenous expression cassette comprising a selectable or screenable marker under the control of a condition-responsive regulatory element. The condition-responsive regulatory element of the exogenous expression cassette of the first cell line is different from the condition-responsive regulatory element of the exogenous expression cassette of the second cell line. For example, in certain aspects, the marker of the exogenous cassette in the first cell line is expressed only if the cell is in a first differentiation state and the marker of the exogenous cassette in the second cell line is expressed only if the cell is in a second differentiation state wherein the first and second differentiation states are distinct.

Cell lines are induced pluripotent stem (iPS) cell lines. Particularly, the iPS cell lines may be essentially free of exogenous viral genetic elements (*e.g.,* free from exogenous retroviral elements), or even more particularly, induced pluripotent stem cells reprogrammed by exogenous episomal vectors, such as OriP-based vectors. It is also disclosed that the cell lines could also be somatic cell lines. In some aspects, an exogenous expression cassette is integrated into the genome of the pluripotent stem cell line(s). The cell line(s) may be, for example, human or mouse cells.

As contemplated in the present invention, the cell lines according to the embodiments could include a wide variety of condition-responsive regulatory elements which control differential expression in a plurality of cell types. Thus, the cells could be used to follow the differential expression of any condition-responsive regulatory element such as tissue-specific promoter in any developmental pathway. For example, a set of cell lines could comprise at least three, four, five, six, seven, nine, ten, 20, 30, 40, 50, 100, 1000, 10, 000, 20,000 (or any range derivable therein) different pluripotent stem cell lines, each comprising a different exogenous expression cassette having a different condition-responsive regulatory element. In a further aspect, cell lines could comprise at least three, four, five, six, seven, nine, ten, 20, 30, 40, 50, 100, 1000, 10,000, 20,000 (or any range derivable therein) different exogenous expression cassettes, each comprising a different condition-responsive regulatory element. At least two exogenous expression cassettes may be comprised in same cells, *i.e.,* the exogenous expression cassette of a first or second cell line may comprise at least two separate exogenous expression cassettes, each comprising a different condition-responsive regulatory element.

For convenience to identify different cell lines, each pluripotent stem cell line of a set may be contained in a separate container different from other cell lines in the set of cell lines. In alternative aspects, two or more different pluripotent stem cell line may be contained in the same container.

Ectopic expression by means of a defined condition-responsive regulatory element such as a promoter or enhancer sequence has the distinct advantage of allowing expression to be regulated in a known spatial and temporal fashion. The power of this aspect partly relies on a collection of condition-responsive regulatory elements, which could respond to endogenous or exogenous factors by controlling or regulating gene expression.

Non-limiting examples of the condition-responsive regulatory elements include a differentiation-responsive promoter, such as a cell-specific promoter which causes expression of a selectable or screenable marker when the pluripotent stem cell of the cell line differentiates to a selected cell lineage or a tissue-specific promoter. Condition-responsive regulatory elements can likewise comprise a drug-responsive regulatory element such as a promoter of a drug metabolizing enzyme, a signaling-responsive promoter which causes expression of a selectable or screenable marker in a cell where a selected drug signaling pathway, drug target or a drug receptor (or a combination thereof) is activated or repressed. As used herein drug refers to a molecule including, but not limited to, small molecules, nucleic acids and proteins or combinations thereof that alter or are candidates for altering a phenotype associated with disease.

Particularly, the condition-responsive regulatory element may comprise a differentiation-specific promoter which causes expression of a selectable or screenable marker when the pluripotent stem cell of the cell line differentiates to a selected cell lineage or tissue type. Therefore, in certain aspects of the invention, each pluripotent stem cell line has a different differentiation-specific promoter that can be used to indicate status of differentiation into different cell types.

In further aspects, a cell line that comprise cell- or tissue-specific expression cassettes (which causes expression of a selectable or screenable marker when the pluripotent stem cell differentiates to a selected cell lineage) may comprise an additional exogenous expression cassette including a selectable or screenable marker under the control of an additional condition-responsive regulatory element such as a drug-responsive regulatory element *(e.g..* a receptor, drug target, drug metabolizing enzyme or signaling pathway-responsive element). Therefore, after differentiating these pluripotent stem cells to a selected cell lineage as indicated by expression of the marker gene under the control of a differentiation-responsive promoter, and optionally after selection of enrichment of desired differentiated cells, the additional expression cassette may be tested for drug response or signaling regulation of the desired differentiated cells. The additional exogenous expression cassette may, in some aspects, be comprised in a transposon system, for example, a piggyBac system.

In certain aspects, the differentiation-responsive promoter could be identified by a bioinformatics analysis of preferentially expressed genes in a selected cell lineage, for example, by transcriptome sequence analysis or genome analysis. Such bioinformatics analysis may involve the use of a data storage device configured to store the transcriptome or genome data, a server configured to query the potential promoter sequence, or a terminal configured to report the promoter analysis result.

Any promoter that is known in the art to be a tissue-specific or cell-specific promoter as well as a promoter responsive to a compound or up-regulation or down-regulation of a cell signaling could be used in aspects of the present invention, such as non-limiting examples listed in Table 1. For example, the promoter could be specific for a selected progenitor cell, such as a neural progenitor-specific promoter, a hematopoietic progenitor-specific promoter, a hepatocyte progenitor-specific promoter, or a cardiac progenitor-specific promoter. In other aspects, the promoter could be specific for a specific degree of differentiation or a selected terminally differentiated cell, such as a hepatocyte, a cardiomyocyte, an endothelial cell, or a neuron. In further aspects, the promoter could be specific for a selected terminally differentiated cell subtype, such as a ventricular cardiomyocyte, an atrial cardiomyocyte, a nodal cardiomyocyte, an arterial endothelial cell, a venous endothelial cell, a lymphatic endothelial cell, a blood-brain barrier endothelial cell, a dopaminergic neuron, a cholinergic neuron, a gabaergic neuron, or a motor neuron.

In an additional aspect, the differentiation-responsive regulatory element may comprise a tissue-specific promoter such as a kidney-specific promoter, a kidney medulla-specific promoter, a kidney cortex-specific promoter, a heart-specific promoter, a pan-cardiac promoter, a heart atria-specific promoter, a heart ventricle-specific promoter, a liver-specific promoter, a neural-specific promoter, a pancreas-specific promoter, a lung-specific promoter, an endothelial-specific promoter, a blood-specific promoter, or an intestine-specific promoter.

An exogenous expression cassette can be inserted into the cell lines (*e.g*., a cell line in a set of lines), wherein the additional expression cassette comprise a drug-responsive regulatory element, such as a promoter of a drug metabolizing enzyme gene. The drug metabolizing enzyme gene may be a cytochrome P450 monooxygenase, N-acetyltransferase, thiopurine methyltransferase, or dihydropyrimidine dehydrogenase. For example, the cytochrome P450 monooxygenase may comprise CYP1A2, CYP2C9, CYP2C18, CYP2C19, CYP2D6, CYP2E1, CYP3A4, or any allelic variants thereof. This additional expression cassette can be operably linked to a marker gene so that any activity causing up or down regulation of the marker gene expression can be observed external to the cells.

In still further aspects, an expression cassette may comprise a drug signaling-specific promoter which causes expression of a marker gene in a cell wherein a selected signaling pathway is up-regulated or down-regulated. Non-limiting examples of a selected drug signaling pathway include tyrosine kinase pathway, heterotrimeric G protein pathway, small GTPase pathway, serine/threonine protein kinase pathway, phosphatase pathway, lipid kinase pathway, hydrolase pathway, cyclic AMP (cAMP)-mediated pathway, cyclic GMP (cGMP)-mediated pathway, phosphatidylinositol-triphosphate (PIP3)-mediated pathway, diacylglycerol (DAG)-mediated pathway, inositol-triphosphate (IP3)-mediated pathway, EF hand domains of calmodulin-mediated signaling pathway, pleckstrin homology domains of the kinase protein AKT-mediated signaling pathway, chromatin regulation signaling pathway, MAPK signaling pathway, apoptosis/autophagy pathway, translational control pathway, cell cycle/checkpoint pathway, DNA damage pathway, Jak/Stat signaling pathway, NF-κB signaling pathway, TGF-β/Smad signaling pathway, lymphocyte signaling pathway, angiogenesis pathway, vesicle trafficking pathway, cytoskeletal signaling pathway, adhesion pathway, glucose metabolism pathway, Wnt/Hedgehog/Notch signaling pathway, stem cell lineage specification pathway, nuclear receptor-mediated pathway, or protein folding and stability signaling pathway.

In certain aspects, a cell line(s) according to the embodiments may comprise an additional exogenous expression cassette including a selectable or screenable marker under the control of a condition-responsive regulatory element such as a drug-responsive regulatory element (e.g., a regulatory element responsive to a drug signaling pathway or from a drug metabolizing enzyme gene). For example, after selection or enrichment of differentiated cells with cell-specific or tissue-specific expression of a selectable or screenable marker, the additional exogenous expression cassette may be used to mark a pathway, a receptor or drug response in such differentiated cells with expression of a different selectable or screenable marker.

For testing drug metabolism or biological response, one or more of the exogenous expression cassettes may further comprise coding sequence for expression of one or more cellular receptors, signaling pathway mediators, transcription factors, druggable targets or cytochrome P450 monooxygenase.

In certain aspects, each exogenous expression cassette, or particularly, each cell-specific or tissue-specific expression cassette in different cells, could include the same selectable or screenable marker, preferably comprised in the same gene delivery system, such as a recombination-mediated vector. Thus, since the cassettes could be similar with the only difference being the condition-responsive elements, the construction of the cassettes and their introduction into aliquots of the same underlying cell line, for example, by recombination, and the assembly of the sets of the cell lines can be performed in parallel in large numbers.

The selectable or screenable marker under the control of different condition-responsive regulatory element could serve as status indicators of these regulatory elements and could aid selection or enrichment of cells that express such markers. For example, the selectable marker could be further defined as an antibiotic resistance gene, such as a gene that confers resistance to puromycin, blastocidin, geneticin, tetracycline, or ampicillin.

The selectable marker may also be an exogenous antigenic epitope, particularly an exogenous surface antigen epitope, such as a mouse CD44 protein or epitope in the human cell lines. For example, the mouse CD44 protein could be used instead of the antibiotic resistance gene for selection or enrichment of desired cells by magnetic cell sorting (with an anti-mouse CD44 antibody) from the mixture. Obviously, one could use any ectopically expressed surface antigenic epitope as the selectable marker.

The screenable marker may be a gene that expresses a cell surface marker, a fluorescent, luminescent or bioluminescent protein, an epitope, chloramphenicol acetyl transferase (CAT), luciferase or β-galactosidase. For instance, the fluorescent protein could be a green fluorescent protein (GFP), red fluorescent protein (RFP), blue fluorescent protein (BFP) or yellow fluorescent protein (YFP), NFAT nitroreductase or a variant thereof Depending on the markers used, the selection or enrichment of cells may comprise fluorescence-activated cell sorting (FACS), CAT assay, luminescence assay or any methods known for an ordinary person in the art to detect or screen for screenable marker expression, in order to select for cells differentiated in a selected cell lineage or in response to a selected condition. An alternative or complementary approach is to test the presence of exogenous transcripts corresponding to the screenable or selectable marker in progeny cells, using conventional methods, such as RT-PCR, *in situ* hybridization, RNA array, or hybridization (*e.g*., Northern blot). In a particular aspect, one or more of the exogenous expression cassettes may include both a selectable and a screenable marker, preferably comprised in a polycistronic transcription unit.

To co-express multiple genes under the same conditional-responsive regulatory element, the expression cassette may comprise a polycistronic transcription unit. Such a polycistronic transcription unit may comprise an internal ribosome entry site (IRES) or a sequence coding for at least one protease cleavage site and/or self-cleaving peptide for polycistronic transcription. For example, there are several self-cleaving peptides such as a viral 2A peptide.

There is also disclosed a method for providing engineered pluripotent stem cells, comprising providing a stem cell line or set of lines of pluripotent stem cells according to the embodiments described above. The method may comprise introducing different exogenous expression cassettes into a single cell line or into respective different cells. For example, the exogenous expression cassettes may be introduced into the cells by a gene delivery system. The gene delivery system could be a vector. Non-limiting examples of a vector include a viral vector, an episomal vector, a transposon-based vector, or a recombinase-mediated cassette exchange vector. In particular, the vector is a recombinase-mediated cassette exchange vector.

For expression of screenable or selectable markers across cell generations, one or more of the exogenous expression cassettes could be integrated or comprised into the genome of the cells in certain aspects of the invention. For example, one or more of the exogenous expression cassettes may be integrated or comprised at a predetermined location or a random location of the genome of the cells. Particularly, the predetermined location may be a Rosa26 locus of the genome of the cells. The Rosa26 locus may be a human Rosa26 locus, particularly a modified locus comprising an exogenous expression cassette as described above. Such an exogenous expression cassette may be particularly flanked by recombination recognition sites for recombination-mediated exchange of cassettes into the Rosa26 locus. In further aspects, one or more cell lines of the set comprise an additional exogenous expression cassette comprised in a transposon system, wherein the additional expression cassette is different from the exogenous expression cassette comprised in a Rosa locus of the same cell.

For providing a useful set of pluripotent stem cells, there may also comprise a method including the steps of: (a) providing an *in vitro* set of cell lines of pluripotent stem cells comprising a condition-responsive exogenous expression cassette *(e.g.,* a cassette under the control of a differentiation-responsive regulatory element that regulates cell- or tissue-specific expression); (b) providing one or more additional expression cassettes under the control of condition-responsive regulatory element, such as a drug-responsive regulatory element of a receptor, drug target, drug metabolizing enzyme or signaling pathway gene; and (c) introducing the one or more additional expression cassettes into the *in vitro* set of cell lines. In a specific aspect, the cell lines are induced pluripotent stem cell lines. Particularly, the iPS cell lines may be essentially free of exogenous retroviral genetic elements, or even more particularly, derived from episomal reprogramming. The cell lines may, in certain aspects, be human or mouse cells.

For example, the cell-specific or tissue-specific exogenous expression cassettes may be comprised in the genome of pluripotent stem cells, particularly, a predetermined location of the genome of pluripotent stem cells, such as a Rosa26 locus.

The cell-specific or tissue-specific exogenous expression cassettes may be introduced into the pluripotent stem cells by a gene delivery system, such as a recombination-mediated cassette exchange vector. Additional expression cassettes may be introduced into pluripotent stem cells by a transposon system, such as a piggyBac transposon system. The cell-specific or tissue-specific exogenous expression cassettes or the additional expression cassettes may comprise a marker gene under the control of respective condition-responsive regulatory elements. Such a marker gene may be a selectable marker, screenable marker, or a combination thereof. In some aspects, the marker gene for all the cell-specific or tissue-specific exogenous expression cassettes are the same. In certain aspects, the marker gene for all the additional expression cassettes are the same. To serve different purposes in some aspects, in the same cell line the marker gene of the cell-specific or tissue-specific exogenous expression cassette may be different from that of the additional expression cassette.

There is also disclosed a method of providing differentiated cells, comprising the steps of: (a) providing an *in vitro* set of stem cell lines of pluripotent stem cells described above or pluripotent stem cells provided in accordance with method described above, wherein the pluripotent stem cells comprise a cell-specific or tissue-specific exogenous expression cassette comprising a selectable or screenable marker under the control of a cell- or tissue-specific regulatory element; and (b) culturing the pluripotent stem cells under a condition to differentiate the pluripotent stem cells, therefore providing differentiated cells.

If the tissue-specific, cell-specific or molecular (e.g., drug) pathway-specific promoter in a pluripotent stem cell line is activated in the stem cell or differentiated daughter cells, either during differentiation or in the terminal differentiation state, the selectable or screenable marker could be expressed and can then be detected or measured. Therefore, it is disclosed that the differentiation method may further comprise selecting or enriching differentiated cells which express the selectable or screenable marker under the control of the cell- or tissue-specific regulatory element. The selection or enrichment may comprise a high-throughput purification, screening or imaging. For example, the selection or enrichment comprises fluorescence-activated cell sorting (FACS), chloramphenicol acetyltransferase (CAT) assay, or luminescence assay.

In certain aspects, there may be provided a method comprising testing an effect of a test compound on the differentiated cells. For example, the test compound is a small molecule drug, a nucleic acid, or a peptide. Such differentiated cells used in this aspect comprise exogenous expression cassettes which include a selectable or screenable marker under the control of a regulatory element responsive to a drug response or signaling pathway activation, alone or in combination with exogenous expression cassettes having cell-specific or tissue-specific regulatory elements.

It is also disclosed thateach different exogenous expression cassette includes a selectable or screenable marker under the control of a promoter of a different drug metabolizing enzyme gene, such as all the variants of a P450 gene. For example, when differentiated to hepatocytes, which could be selected or enriched using the expression of a selectable or screenable marker under the control of a hepatocyte-specific promoter, the differentiated cells could then be tested for the spectrum of drug response such as P450 responses.

In a further aspect, there may be provided a method of testing a differentiation condition, comprising the steps of: (a) providing a pluripotent stem cell comprising an exogenous expression cassette including a selectable or screenable marker under the control of a condition-responsive regulatory element which causes expression of the selectable or screenable marker when the pluripotent stem cell differentiates to a selected cell lineage or tissue; and (b) culturing the pluripotent stem cell under a test condition and determining whether the test condition differentiate the pluripotent stem cell to a selected cell lineage or tissue, wherein if differentiated to the selected cell lineage or tissue, progeny cells of the pluripotent stem cell express the selectable or screenable marker. This method could be used to screen novel conditions that could be used to provide cells of a cell type by differentiation. The test condition may be a drug, a peptide, a nucleic acid, or a culture condition. This method may also be used for other aspects of programming, like transdifferentiation or dedifferentiation in a similar manner. The method may further comprise further comprising selecting or enriching differentiated cells which express the selectable or screenable marker under the control of the condition-responsive regulatory element.

There may comprise a method of testing a compound for its effect on differentiation of specific cell or tissue types, comprising the steps of: (a) providing a pluripotent stem cell comprising an exogenous expression cassette including a selectable or screenable marker under the control of a condition-responsive regulatory element which causes expression of the selectable or screenable marker when the pluripotent stem cell differentiates to a selected cell lineage or tissue; and (b) culturing the pluripotent stem cell under a differentiation condition in the presence of a test compound, wherein the differentiation condition is capable of differentiating pluripotent stem cells into the selected cell lineage or tissue *per se*; and c) determining the expression of the selectable or screenable marker for the effect of the testing compound on the differentiation of the pluripotent stem cell to the selected cell lineage or tissue.

In a further aspect a method of testing a compound (e.g., a drug) is provided, comprising the steps of: (a) providing a pluripotent stem cell comprising (i) a first exogenous expression cassette comprising a selectable or screenable marker under the control of a differentiation-responsive regulatory element and (ii) a second exogenous expression cassette comprising a screenable marker under the control of a drug-responsive regulatory element, and culturing the cell under differentiation conditions sufficient to cause expression of the first expression cassette; (b) contacting the cell with a drug; and (c) determining a response to the drug by determining expression of the second expression cassette. Such
a method may comprise testing a plurality of compounds such as at least about 10, 100, 1,0000, 10,000 or more compounds.

Embodiments discussed in the context of methods and/or compositions of the invention may be employed with respect to any other method or composition described herein. Thus, an embodiment pertaining to one method or composition may be applied to other methods and compositions of the invention as well.

As used herein the terms "encode" or "encoding" with reference to a nucleic acid are used to make the invention readily understandable by the skilled artisan however these terms may be used interchangeably with "comprise" or "comprising" respectively.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating preferred embodiments of the invention, are given by way of illustration.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

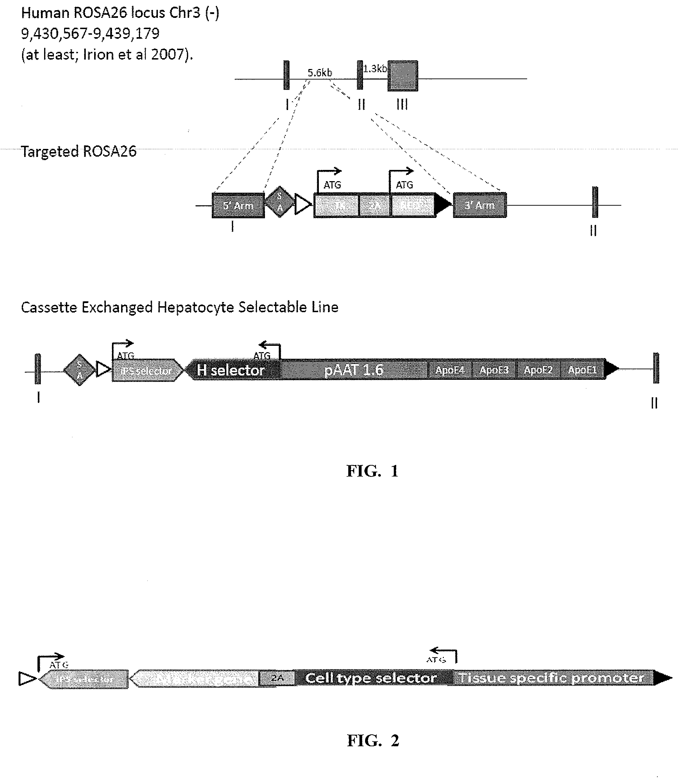

**FIG. 1****.** An illustrative exemplary embodiment of a Rosa26 targeting cassette (upper) and a cassette-exchanged hepatocyte selectable line (lower). Upper: inserted in between intron I and II in the native Rosa 26 locus on human chromosome 3, the Rosa 26 targeting cassette included, in 5' to 3' sequence, a 5' homologous arm for targeting, a spacer, a recombinase recognition site (white triangle), a protein coding sequence from the thymidine kinase gene beginning with an ATG to start transcription, a 2A sequence, a second protein coding sequence for an antibiotic resistance gene for resistance to neomycin, a second recombinase recognition site (black triangle) and a 3' homologous arm. Lower: the elements of an exogenous genetic construct in a secondary engineered iPS line constructed for selection of hepatocytes are shown. The secondary iPS line is made from the basal Rosa 26 iPS line comprising the Rosa26 targeting cassette. The genetic construct was assembled which contained two expression cassettes, one cassette to permit selection of the desired recombinant event, and one cassette to permit tissue specific selection of the desired tissue type, *i.e*., hepatocytes. At the 5' end of the construct, there was the left recombination recognition site, followed by the protein coding sequence for another antibiotic resistance, designed herein as the iPS selector. This coding sequence is driven by the native Rosa 26 promoter to permit successful desired recombinant cells to be identified by resistance to the antibiotic for which the iPS selector confers resistance. Also in the construct, oriented in the opposite direction, is a construct including the promoter of alpha-1-antitrypsin (pAAT), which drives the expression of a second antibiotic selection gene, this one to be used to select cells when the cells have differentiated into hepatocytes. In this particular construct, there are also several enhancer elements (designated as ApoE1-4).
**FIG. 2** is an example of the common format of design of the genetic constructions to go into the iPS lines of the collection. For each insertion, there is an iPS selector which permits selection of the desired recombinant insertion. For each insertion, there is a tissue specific promoter, the promoters being different in different elements of the set, but each of the promoters selected for tissue specific expression. The tissue specific expression will be in some instances an organ, *e.g*., pan cardiac, in some instances an organ subtype, *e.g.,* atrial cell, in some cases a body wide cell type, *e.g.,* endothelial cell, or in some instances a level of differentiation, *e.g*., a cardiac progenitor. The tissue specific promoter actuates expression of a second gene for resistance to a second antibiotic resistance gene, labeled a cell type selector. A marker gene, such as a fluorescent protein, luciferase, a proprietary marker system, such as HaloTag or SNAP, is linked in expression to the cell type selector by a 2A linker, which works to express two distinct proteins driven by a common promoter.

### DESCRIPTION OF ILLUSTRATIVE EMBODIMENTS

The instant invention overcomes several major problems with current technologies by providing methods and compositions related to engineered pluripotent stem cells that can be used to study any biological response in the human body. These engineered pluripotent cells provide a tool kit with a wide range of applications not adequately addressed by current technology. For example, cells are provided comprising two or more exogenous expression cassettes each comprising a selectable or screenable marker under the control of different condition-responsive regulatory elements. These cells allow for simultaneous testing of two or more different conditions that are interrogated by the expression of screenable or selectable markers from the cassettes. Thus, the engineered cells allow for rapid assessment of, for example, efficacy and toxicity of new drug candidates. In further aspects, the cells may be employ to develop and optimize directed cell differentiation protocols.

In one example, a pluripotent stem cell according to the embodiments comprises at least two exogenous expression cassettes. The first cassette includes a condition-responsive regulatory element that provides expression in response to a compound of interest, such as a drug candidate. The second cassette then includes a condition-responsive regulatory element providing expression when the cell differentiates into a selected cell lineage. Accordingly, the cell line can be used to test cellular response to a drug candidate by culturing the cell line to differentiate cells into the lineage of interest. Expression from the second cassette is thus indicative of differentiation into the lineage of interest. Differentiated cells are then contacted with the drug candidate and expression from the first cassette is determined to assess response to the drug candidate. Alternatively or additionally, cells can be selected based on expression from the second cassette to provide an essentially pure population of cells of a lineage of interest for testing of the drug a candidate. Thus, the cells provide a lineage-specific readout of the effects of candidate drug molecules.

In a related aspect, a pluripotent cell line according to the embodiments comprises a first exogenous expression cassette with a condition-responsive regulatory element that provides expression in response to a compound of interest *(e.g.,* a drug candidate). From this cell line a panel of lines can be generated, each comprising at least a second exogenous expression cassette (*e.g*., a cassette comprised in a transposon system) with a condition-responsive regulatory element that is active only when cells differentiate into a selected lineage or cell type of interest. Thus, cell lines in the panel can be differentiated into an array of cell lineages. In each case, the differentiation status of the cells can be confirmed, or the cells selected, based on expression from the second expression cassette. The effect of a compound of interest can thereby be determined on a whole range of different cell lineages by contacting the differentiated cells with the compound and detecting expression of the first expression cassette. In this case, each cell line in the panel is able to provide information regarding a different differentiated cell type. As a whole, such a panel could provide information on drug effect and/or toxicity for essentially all of the cell lineages in an organ or tissue of interest.

Conversely, a panel of cells can be generated using as a base a cell line comprising an expression cassette that provides expression a particular cell lineage. A panel of such cells is then generated with each line comprising a further expression cassette that provides expression upon activation a pathway of interest. Such cells can then be differentiated to the cell lineage of interest, as confirmed (or selected) by expression from the base expression cassette. The various differentiated cells in the panel are then treated with a drug or a panel of drugs and expression from the further expression cassettes is assessed to determine the effect of the drug(s) on a range of different metabolic pathways. In this example, an array of metabolic pathways in a particular cell type can be assayed simultaneously to provide a complete picture of the effect of a drug candidate or panel of candidates on the particular cell type.

The engineered pluripotent stem cells and panels of stem cells of the embodiments thus provide a highly adaptable, high throughput system, for interrogating cellular response in any type cell or tissue and at virtually any stage of differentiation. The cells can be used, for example, to simultaneously test the effect of drug candidates on a plurality of metabolic pathways and/or in a plurality of different cell types. Likewise the engineered cells can be used to test and refine differentiation conditions for producing cells types of interest. In this case, the ability to simultaneously integrate the appearance of multiple cell lineages in a population allows differentiation conditions to be refine to either eliminate undesirable cell lineages or to enhance the proportion of a lineage of interest. Moreover these cells can be used to develop differentiation protocols that provide a population of differentiated cells having a desired proportion of different cell types. The engineered cells, thus, constitute a new and powerful tool to address lineage specific differentiation and cellular response that was not previously available.

Further embodiments and advantages of the invention are described below.

### I. Definitions

"Programming" is a process that changes a cell to form progeny of at least one new cell type, either in culture or *in vivo,* than it would have under the same conditions without programming. This means that after sufficient proliferation, a measurable proportion of progeny having phenotypic characteristics of the new cell type if essentially no such progeny could form before programming; alternatively, the proportion having characteristics of the new cell type is measurably more than before programming. This process includes differentiation, dedifferentiation and transdifferentiation. "Differentiation" is the process by which a less specialized cell becomes a more specialized cell type. "Dedifferentiation" is a cellular process in which a partially or terminally differentiated cell reverts to an earlier developmental stage, such as pluripotency or multipotency. "Transdifferentiation" is a process of transforming one differentiated cell type into another differentiated cell type. Under certain conditions, the proportion of progeny with characteristics of the new cell type may be at least about 1%, 5%, 25% or more in the order of increasing preference.

"Reprogramming" is a process that confers on a cell a measurably increased capacity to form progeny of at least one new cell type, either in culture or *in vivo,* than it would have under the same conditions without reprogramming. Dedifferentiation may include reprogramming. More specifically, reprogramming is a process that confers on a somatic cell a pluripotent potential. This means that after sufficient proliferation, a measurable proportion of progeny having phenotypic characteristics of the new cell type if essentially no such progeny could form before reprogramming; otherwise, the proportion having characteristics of the new cell type is measurably more than before reprogramming. Under certain conditions, the proportion of progeny with characteristics of the new cell type may be at least about 1%, 5%, 25% or more in the order of increasing preference.

The term "exogenous," when used in relation to a protein, gene, nucleic acid, or polynucleotide in a cell or organism refers to a protein, gene, nucleic acid, or polynucleotide which has been introduced into the cell or organism by artificial means, or in relation to a cell refers to a cell which was isolated and subsequently introduced to other cells or to an organism by artificial means. An exogenous nucleic acid may be from a different organism or cell, or it may be one or more additional copies of a nucleic acid which occurs naturally within the organism or cell. An exogenous cell may be from a different organism, or it may be from the same organism. By way of a non-limiting example, an exogenous nucleic acid is in a chromosomal location different from that of natural cells, or is otherwise flanked by a different nucleic acid sequence than that found in nature.

By "expression construct" or "expression cassette" is meant a nucleic acid molecule that is capable of directing transcription. An expression construct includes, at the least, one or more transcriptional control elements (such as promoters, enhancers or a structure functionally equivalent thereof) that direct gene expression in one or more desired cell types, tissues or organs. Additional elements, such as a transcription termination signal, may also be included.

A "vector" or "construct" (sometimes referred to as gene delivery system or gene transfer "vehicle") refers to a macromolecule or complex of molecules comprising a polynucleotide to be delivered to a host cell, either *in vitro* or *in vivo.*

A "plasmid", a common type of a vector, is an extra-chromosomal DNA molecule separate from the chromosomal DNA which is capable of replicating independently of the chromosomal DNA. In certain cases, it is circular and double-stranded.

The term "corresponds to" is used herein to mean that a polynucleotide sequence is homologous (*i.e.,* is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

A "gene," "polynucleotide," "coding region," "sequence, " "segment," "fragment," or "transgene" which "encodes" a particular protein, is a nucleic acid molecule which is transcribed and optionally also translated into a gene product, *e.g.,* a polypeptide, *in vitro* or *in vivo* when placed under the control of appropriate regulatory sequences. The coding region may be present in either a cDNA, genomic DNA, or RNA form. When present in a DNA form, the nucleic acid molecule may be single-stranded (*i.e.,* the sense strand) or double-stranded. The boundaries of a coding region are determined by a start codon at the 5' (amino) terminus and a translation stop codon at the 3' (carboxy) terminus. A gene can include, but is not limited to, cDNA from prokaryotic or eukaryotic mRNA, genomic DNA sequences from prokaryotic or eukaryotic DNA, and synthetic DNA sequences. A transcription termination sequence will usually be located 3' to the gene sequence.

The term "control elements" refers collectively to promoter regions, polyadenylation signals, transcription termination sequences, upstream regulatory domains, origins of replication, internal ribosome entry sites ("IRES"), enhancers, splice junctions, and the like, which collectively provide for the replication, transcription, post-transcriptional processing and translation of a coding sequence in a recipient cell. Not all of these control elements need always be present so long as the selected coding sequence is capable of being replicated, transcribed and translated in an appropriate host cell.

The term "promoter" is used herein in its ordinary sense to refer to a nucleotide region comprising a DNA regulatory sequence, wherein the regulatory sequence is derived from a gene which is capable of binding RNA polymerase and initiating transcription of a downstream (3' direction) coding sequence.

By "enhancer" is meant a nucleic acid sequence that, when positioned proximate to a promoter, confers increased transcription activity relative to the transcription activity resulting from the promoter in the absence of the enhancer domain.

By "operably linked" with reference to nucleic acid molecules is meant that two or more nucleic acid molecules (*e.g.,* a nucleic acid molecule to be transcribed, a promoter, and an enhancer element) are connected in such a way as to permit transcription of the nucleic acid molecule. "Operably linked" with reference to peptide and/or polypeptide molecules is meant that two or more peptide and/or polypeptide molecules are connected in such a way as to yield a single polypeptide chain, *i.e.,* a fusion polypeptide, having at least one property of each peptide and/or polypeptide component of the fusion. The fusion polypeptide is preferably chimeric, *i.e*., composed of heterologous molecules.

"Homology" refers to the percent of identity between two polynucleotides or two polypeptides. The correspondence between one sequence and another can be determined by techniques known in the art. For example, homology can be determined by a direct comparison of the sequence information between two polypeptide molecules by aligning the sequence information and using readily available computer programs. Alternatively, homology can be determined by hybridization of polynucleotides under conditions which form stable duplexes between homologous regions, followed by digestion with single strand-specific nuclease(s), and size determination of the digested fragments. Two DNA, or two polypeptide, sequences are "substantially homologous" to each other when at least about 80%, preferably at least about 90%, and most preferably at least about 95% of the nucleotides, or amino acids, respectively match over a defined length of the molecules, as determined using the methods above.

The term "cell" is herein used in its broadest sense in the art and refers to a living body which is a structural unit of tissue of a multicellular organism, is surrounded by a membrane structure which isolates it from the outside, has the capability of self replicating, and has genetic information and a mechanism for expressing it. Cells used herein may be naturally-occurring cells, synthetic cells, or artificially modified cells (*e.g*., fusion cells, genetically modified cells, *etc*.).

As used herein, the term "stem cell" refers to a cell capable of giving rising to at least one type of a more specialized cell. A stem cell has the ability to self-renew, *i.e.,* to go through numerous cycles of cell division while maintaining the undifferentiated state, and has potency, *i.e.,* the capacity to differentiate into specialized cell types. Typically, stem cells can regenerate an injured tissue. Stem cells herein may be, but are not limited to, embryonic stem (ES) cells, induced pluripotent stem (iPS) cells, or tissue stem cells (also called tissue-specific stem cell, or somatic stem cell). Any artificially produced cell which can have the above-described abilities (*e.g*., fusion cells, reprogrammed cells, or the like used herein) may be a stem cell.

"Embryonic stem (ES) cells" are pluripotent stem cells derived from early embryos. An ES cell was first established in 1981, which has also been applied to production of knockout mice since 1989. In 1998, a human ES cell was established, which is currently becoming available for regenerative medicine.

Unlike ES cells, tissue stem cells have a limited differentiation potential. Tissue stem cells are present at particular locations in tissues and have an undifferentiated intracellular structure. Therefore, the pluripotency of tissue stem cells is typically low. Tissue stem cells have a higher nucleus/cytoplasm ratio and have few intracellular organelles. Most tissue stem cells have low pluripotency, a long cell cycle, and proliferative ability beyond the life of the individual. Tissue stem cells are separated into categories, based on the sites from which the cells are derived, such as the dermal system, the digestive system, the bone marrow system, the nervous system, and the like. Tissue stem cells in the dermal system include epidermal stem cells, hair follicle stem cells, and the like. Tissue stem cells in the digestive system include pancreatic (common) stem cells, liver stem cells, and the like. Tissue stem cells in the bone marrow system include hematopoietic stem cells, mesenchymal stem cells, and the like. Tissue stem cells in the nervous system include neural stem cells, retinal stem cells, and the like.

"Induced pluripotent stem cells," commonly abbreviated as iPS cells or iPSCs, refer to a type of pluripotent stem cell artificially prepared from a non-pluripotent cell, typically an adult somatic cell, or terminally differentiated cell, such as fibroblast, a hematopoietic cell, a myocyte, a neuron, an epidermal cell, or the like, by inserting certain genes, referred to as reprogramming factors.

"Pluripotency" refers to a stem cell that has the potential to differentiate into all cells constituting one or more tissues or organs, or preferably, any of the three germ layers: endoderm (interior stomach lining, gastrointestinal tract, the lungs), mesoderm (muscle, bone, blood, urogenital), or ectoderm (epidermal tissues and nervous system). "Pluripotent stem cells" used herein refer to cells that can differentiate into cells derived from any of the three germ layers, for example, direct descendants of totipotent cells or induced pluripotent cells.

As used herein "totipotent stem cells" refers to cells has the ability to differentiate into all cells constituting an organism, such as cells that are produced from the fusion of an egg and sperm cell. Cells produced by the first few divisions of the fertilized egg are also totipotent. These cells can differentiate into embryonic and extraembryonic cell types. Pluripotent stem cells can give rise to any fetal or adult cell type. However, alone they cannot develop into a fetal or adult animal because they lack the potential to contribute to extraembryonic tissue, such as the placenta: Totipotent cells are not encompassed by the present invention.

In contrast, many progenitor cells are multipotent stem cells, *i.e.,* they are capable of differentiating into a limited number of cell fates. Multipotent progenitor cells can give rise to several other cell types, but those types are limited in number. An example of a multipotent stem cell is a hematopoietic cell - a blood stem cell that can develop into several types of blood cells, but cannot develop into brain cells or other types of cells. At the end of the long series of cell divisions that form the embryo are cells that are terminally differentiated, or that are considered to be permanently committed to a specific function.

As used herein, the term "somatic cell" refers to any cell other than germ cells, such as an egg, a sperm, or the like, which does not directly transfer its DNA to the next generation. Typically, somatic cells have limited or no pluripotency. Somatic cells used herein may be naturally-occurring, synthetic, or genetically modified.

### II. Sources of Cells

In the context of the present disclosure, there are disclosed methods and compositions for providing an *in vitro* set of cell lines comprising stem cells or differentiated cells that comprise different exogenous expression cassettes. The cells mav be stem cells, including but are not limited to, embryonic stem cells, fetal stem cells, or adult stem cells. The cells may be any somatic cells. Thus, it will be recognized that, in certain aspects, cell lines are made without destruction of human embryos.

### B. Stem Cells

Stem cells are cells found in most, if not all, multi-cellular organisms. They are characterized by the ability to renew themselves through mitotic cell division and differentiating into a diverse range of specialized cell types. The two broad types of mammalian stem cells are: embryonic stem cells that are found in blastocysts, and adult stem cells that are found in adult tissues. In a developing embryo, stem cells can differentiate into all of the specialized embryonic tissues. In adult organisms, stem cells and progenitor cells act as a repair system for the body, replenishing specialized cells, but also maintain the normal turnover of regenerative organs, such as blood, skin or intestinal tissues.

Human embryonic stem cells (ESCs) and induced pluripotent stem cells (iPSC) are capable of long-term proliferation *in vitro,* while retaining the potential to differentiate into all cell types of the body, including hepatocytes. Thus these cells could potentially provide an unlimited supply of various patient-specific differentiated cells such as functional hepatocytes for research, drug development and transplantation therapies.

### 2. Embryonic stem cells

Embryonic stem cell lines (ES cell lines) are cultures of cells derived from the epiblast tissue of the inner cell mass (ICM) of a blastocyst or earlier morula stage embryos. A blastocyst is an early stage embryo-approximately four to five days old in humans and consisting of 50-150 cells. ES cells are pluripotent and give rise during development to all derivatives of the three primary germ layers: ectoderm, endoderm and mesoderm. In other words, they can develop into each of the more than 200 cell types of the adult body when given sufficient and necessary stimulation for a specific cell type. They do not contribute to the extra-embryonic membranes or the placenta.

Nearly all research to date has taken place using mouse embryonic stem cells (mES) or human embryonic stem cells (hES). Both have the essential stem cell characteristics, yet they require very different environments in order to maintain an undifferentiated state. Mouse ES cells may be grown on a layer of gelatin and require the presence of Leukemia Inhibitory Factor (LIF). Human ES cells could be grown on a feeder layer of mouse embryonic fibroblasts (MEFs) and often require the presence of basic Fibroblast Growth Factor (bFGF or FGF-2). Without optimal culture conditions or genetic manipulation (*Chambers et al.,* 2003), embryonic stem cells will rapidly differentiate.

A human embryonic stem cell may be also defined by the presence of several transcription factors and cell surface proteins. The transcription factors Oct-4, Nanog, and Sox-2 form the core regulatory network that ensures the suppression of genes that lead to differentiation and the maintenance of pluripotency (*Boyer et al.,* 2005). The cell surface antigens most commonly used to identify pluripotent stem cells include the glycolipids SSEA3 and SSEA4 and the keratan sulfate antigens Tra-1-60 and Tra-1-81.

Methods for obtaining mouse ES cells are well known. In one method, a preimplantation blastocyst from the 129 strain of mice is treated with mouse antiserum to remove the trophoectoderm, and the inner cell mass is cultured on a feeder cell layer of chemically inactivated mouse embryonic fibroblasts in medium containing fetal calf serum. Colonies of undifferentiated ES cells that develop are subcultured on mouse embryonic fibroblast feeder layers in the presence of fetal calf serum to produce populations of ES cells. In some methods, mouse ES cells can be grown in the absence of a feeder layer by adding the cytokine leukemia inhibitory factor (LIF) to serum-containing culture medium (Smith, 2000). In other methods, mouse ES cells can be grown in serum-free medium in the presence of bone morphogenetic protein and LIF (Ying *et al.,* 2003).

Human ES cells can be obtained from blastocysts using previously described methods (Thomson *et al.,* 1995; Thomson *et al.,* 1998; Thomson and Marshall, 1998; Reubinoff *et al,* 2000.) In one method, day-5 human blastocysts are exposed to rabbit antihuman spleen cell antiserum, then exposed to a 1:5 dilution of Guinea pig complement to lyse trophectoderm cells. After removing the lysed trophectoderm cells from the intact inner cell mass, the inner cell mass is cultured on a feeder layer of gamma-inactivated mouse embryonic fibroblasts and in the presence of fetal bovine serum. After 9 to 15 days, clumps of cells derived from the inner cell mass can be chemically (*i.e*., exposed to trypsin) or mechanically dissociated and replated in fresh medium containing fetal bovine serum and a feeder layer of mouse embryonic fibroblasts. Upon further proliferation, colonies having undifferentiated morphology are selected by micropipette, mechanically dissociated into clumps, and replated (see U.S. Patent No. 6,833,269). ES-like morphology is characterized as compact colonies with apparently high nucleus to cytoplasm ratio and prominent nucleoli. Resulting ES cells can be routinely passaged by brief trypsinization or by selection of individual colonies by micropipette. In some methods, human ES cells can be grown without serum by culturing the ES cells on a feeder layer of fibroblasts in the presence of basic fibroblast growth factor (Amit *et al.,* 2000). In other methods, human ES cells can be grown without a feeder cell layer by culturing the cells on a protein matrix such as Matrigel™ or laminin in the presence of "conditioned" medium containing basic fibroblast growth factor (Xu *et al.,* 2001). The medium is previously conditioned by coculturing with fibroblasts.

Methods for the isolation of rhesus monkey and common marmoset ES cells are also known (Thomson, and Marshall, 1998; Thomson *et al.,* 1995; Thomson and Odorico, 2000).

Another source of ES cells are established ES cell lines. Various mouse cell lines and human ES cell lines are known and conditions for their growth and propagation have been defined. For example, the mouse CGR8 cell line was established from the inner cell mass of mouse strain 129 embryos, and cultures of CGR8 cells can be grown in the presence of LIF without feeder layers. As a further example, human ES cell lines H1, H7, H9, H13 and H14 were established by Thompson *et al.* In addition, subclones H9.1 and H9.2 of the H9 line have been developed. It is disclosed that virtually any ES or stem cell line known in the art and may be used in the context of the present disclosure, such as, *e.g.,* those described in Yu and Thompson, 2008.

The source of ES cells for use in connection with the present disclosure can be a blastocyst, cells derived from culturing the inner cell mass of a blastocyst, or cells obtained from cultures of established cell lines. Thus, as used herein, the term "ES cells" can refer to inner cell mass cells of a blastocyst, ES cells obtained from cultures of inner mass cells, and ES cells obtained from cultures of ES cell lines.

### 3. Induced pluripotent stem cells

Induced pluripotent stem (iPS) cells are cells which have the characteristics of ES cells but are obtained by the reprogramming of differentiated somatic cells. Induced pluripotent stem cells have been obtained by various methods. In one method, adult human dermal fibroblasts are transformed with transcription factors Oct4, Sox2, c-Myc and Klf4 using retroviral transduction (Takahashi *et al.,* 2007). The transformed cells are plated on SNL feeder cells (a mouse cell fibroblast cell line that produces LIF) in medium supplemented with basic fibroblast growth factor (bFGF). After approximately 25 days, colonies resembling human ES cell colonies appear in culture. The ES cell-like colonies are picked and expanded on feeder cells in the presence of bFGF.

Based on cell characteristics, cells of the ES cell-like colonies are induced pluripotent stem cells. The induced pluripotent stem cells are morphologically similar to human ES cells, and express various human ES cell markers. Also, when grown under conditions that are known to result in differentiation of human ES cells, the induced pluripotent stem cells differentiate accordingly. For example, the induced pluripotent stem cells can differentiate into cells having neuronal structures and neuronal markers. It is anticipated that virtually any iPS cells or cell lines may be used with the present invention, including, *e.g*., those described in Yu and Thompson, 2008.

In another method, human fetal or newborn fibroblasts are transformed with four genes, Oct4, Sox2, Nanog and Lin28 using lentivirus transduction (Yu *et al.,* 2007). At 12-20 days post infection, colonies with human ES cell morphology become visible. The colonies are picked and expanded. The induced pluripotent stem cells making up the colonies are morphologically similar to human ES cells, express various human ES cell markers, and form teratomas having neural tissue, cartilage and gut epithelium after injection into mice.

Methods of preparing induced pluripotent stem cells from mouse are also known (Takahashi and Yamanaka, 2006). Induction of iPS cells typically require the expression of or exposure to at least one member from Sox family and at least one member from Oct family. Sox and Oct are thought to be central to the transcriptional regulatory hierarchy that specifies ES cell identity. For example, Sox may be Sox-1, Sox-2, Sox-3, Sox-15, or Sox-18; Oct may be Oct-4. Additional factors may increase the reprogramming efficiency, like Nanog, Lin28, Klf4, or c-Myc; specific sets of reprogramming factors may be a set comprising Sox-2, Oct-4, Nanog and, optionally, Lin-28; or comprising Sox-2, Oct4, Klf and, optionally, c-Myc.

iPS cells, like ES cells, have characteristic antigens that can be identified or confirmed by immunohistochemistry or flow cytometry, using antibodies for SSEA-1, SSEA-3 and SSEA-4 (Developmental Studies Hybridoma Bank, National Institute of Child Health and Human Development, Bethesda Md.), and TRA-1-60 and TRA-1-81 (Andrews *et al.,* 1987). Pluripotency of embryonic stem cells can be confirmed by injecting approximately 0.5-10 X 10⁶ cells into the rear leg muscles of 8-12 week old male SCID mice. Teratomas develop that demonstrate at least one cell type of each of the three germ layers.

It is also disclosed that iPS cells are made from reprogramming somatic cells using reprogramming factors comprising an Oct family member and a Sox family member, such as Oct4 and Sox2 in combination with Klf or Nanog as described above. The somatic cell for reprogramming may be any somatic cell that can be induced to pluripotency, such as a fibroblast, a keratinocyte, a hematopoietic cell, a mesenchymal cell, a liver cell, a stomach cell, or a P cell. In a certain aspect, T cells may also be used as source of somatic cells for reprogramming (see U.S. Application No. 12/478,154, or RNA transfection (see U.S. Application No. 12/735,060).

Reprogramming factors may be expressed from exogenous expression cassettes comprised in one or more vectors, such as an integrating vector or an episomal vector. Reprogramming proteins could be introduced directly into somatic cells by protein transduction (see U.S. Application No. 61/172,079,).

A particular type of cell source for use in certain aspects as disclosed herein is an iPS cell line made by episomal reprogramming, *e.g*., an EBV element-based system (see US Publication No. 2010/0003757; Yu et al., 2009). Episomal reprogramming results in iPS cells genetically identical to the cells of the patient who donated the cells which were reprogrammed, and no foreign genetic material will be integrated into the genome of the reprogrammed cells by this method. The episomal reprogramming method can be done under fully defined conditions and is reliable, efficient and well defined. iPS lines made by episomal reprogramming can be differentiated into any desired lineage and reproduce infinitely in culture.

### 4. Embryonic stem cells derived by somatic cell nuclear transfer

It is also disclosed thatpluripotent stem cells can be prepared by means of somatic cell nuclear transfer, in which a donor nucleus is transferred into a spindle-free oocyte. Stem cells produced by nuclear transfer are genetically identical to the donor nuclei. In one method, donor fibroblast nuclei from skin fibroblasts of a rhesus macaque are introduced into the cytoplasm of spindle-free, mature metaphase II rhesus macaque oocytes by electrofusion (Byrne *et al.,* 2007). The fused oocytes are activated by exposure to ionomycin, and then incubated until the blastocyst stage. The inner cell mass of selected blastocysts are then cultured to produce embryonic stem cell lines. The embryonic stem cell lines show normal ES cell morphology, express various ES cell markers, and differentiate into multiple cell types both *in vitro* and *in vivo.* As used herein, the term "ES cells" refers to embryonic stem cells derived from embryos containing fertilized nuclei. ES cells are distinguished from embryonic stem cells produced by nuclear transfer, which are referred to as "embryonic stem cells derived by somatic cell nuclear transfer."

### 5. Other stem cells

Fetal stem cells are cells with self-renewal capability and pluripotent differentiation potential. They can be isolated and expanded from fetal cytotrophoblast cells (European Patent EP0412700) and chorionic villi, amniotic fluid and the placenta (WO/2003/042405). Cell surface markers of fetal stem cells include CD117/c-kit⁺, SSEA3⁺, SSEA4⁺ and SSEA1⁻.

Somatic stem cells have been identified in most organ tissues. The best characterized is the hematopoietic stem cell. This is a mesoderm-derived cell that has been purified based on cell surface markers and functional characteristics. The hematopoietic stem cell, isolated from bone marrow, blood, cord blood, fetal liver and yolk sac, is the progenitor cell that reinitiates hematopoiesis for the life of a recipient and generates multiple hematopoietic lineages (see U.S. Pat. No. 5,635,387; 5,460,964; 5,677,136; 5,750,397; 5,759,793; 5,681,599; 5,716,827; Hill *et al.,* 1996).

When transplanted into lethally irradiated animals or humans, hematopoietic stem cells can repopulate the erythroid, neutrophil-macrophage, megakaryocyte and lymphoid hematopoietic cell pool. *In vitro,* hematopoietic stem cells can be induced to undergo at least some self-renewing cell divisions and can be induced to differentiate to the same lineages as is seen *in vivo.* Therefore, this cell fulfills the criteria of a stem cell.

The next best characterized is the mesenchymal stem cells (MSC), originally derived from the embryonic mesoderm and isolated from adult bone marrow, can differentiate to form muscle, bone, cartilage, fat, marrow stroma, and tendon. During embryogenesis, the mesoderm develops into limb-bud mesoderm, tissue that generates bone, cartilage, fat, skeletal muscle and possibly endothelium. Mesoderm also differentiates to visceral mesoderm, which can give rise to cardiac muscle, smooth muscle, or blood islands consisting of endothelium and hematopoietic progenitor cells. Primitive mesodermal or mesenchymal stem cells, therefore, could provide a source for a number of cell and tissue types. A number of mesenchymal stem cells have been isolated (see, for example, U.S. Pat. No. 5,486,359; 5,827,735; 5,811,094; 5,736,396; U.S. Pat. No. 5,837,539; 5,837,670; 5,827,740; Jaiswal *et al.,* 1997; Cassiede *et al.,* 1996; Johnstone *et al.,* 1998; Yoo *et al.,* 1998; Gronthos, 1994; Makino *et al.,* 1999).

Of the many mesenchymal stem cells that have been described, all have demonstrated limited differentiation to form only those differentiated cells generally considered to be of mesenchymal origin. To date, the most multipotent mesenchymal stem cell expresses the SH2⁺ SH4⁺ CD29⁺ CD44⁺ CD71⁺ CD90⁺ CD106⁺ CD120a⁺ CD124⁺ CD14- CD34⁻ CD45⁻ phenotype.

Other stem cells have been identified, including gastrointestinal stem cells, epidermal stem cells, neural and hepatic stem cells, also termed oval cells (Potten, 1998; Watt, 1997; Alison *et al,* 1998).

It is disclosed that the stem cells useful for the method described herein include but not limited to embryonic stem cells, induced plurpotent stem cells, mesenchymal stem cells, bone-marrow derived stem cells, hematopoietic stem cells, chrondrocytes progenitor cells, epidermal stem cells, gastrointestinal stem cells, neural stem cells, hepatic stem cells adipose-derived mesenchymal stem cells, pancreatic progenitor cells, hair follicular stem cells, endothelial progenitor cells and smooth muscle progenitor cells.

It is disclosed that the stem cells used for the method described herein may isolated from umbilical cord, placenta, amniotic fluid, chorion villi, blastocysts, bone marrow, adipose tissue, brain, peripheral blood, the gastrointestinal tract, cord blood, blood vessels, skeletal muscle, skin, liver and menstrual blood. Stem cells prepared in the menstrual blood are called endometrial regenerative cells (Medistem Inc.).

One ordinary skilled artisan in the art can locate, isolate and expand such stem cells. The detailed procedures for the isolation of human stem cells from various sources are described in Current Protocols in Stem Cell Biology (2007) .

Alternatively, commercial kits and isolation systems can be used. For example, the BD FACSAria cell sorting system, BD IMag magnetic cell separation system, and BD IMag mouse hematopoietic progenitor cell enrichment set from BD Biosciences. Methods of isolating and culturing stem cells from various sources are also described in 5,486,359, 6,991,897, 7,015,037, 7,422,736, 7,410,798, 7,410,773, 7,399,632.

### C. Somatic cells

In the context of the present disclosure, there are also disclosed engineered somatic cell lines having the exogenous expression cassettes. The somatic cell lines may be used in methods of transdifferentiation, *i.e.,* the direct conversion of one somatic cell type into another, *e.g*., deriving hepatocytes from other somatic cells.

However, the human somatic cells may be limited in supply, especially from living donors. In certain aspects to provide a unlimited supply of starting cells, somatic cells may be immortalized by introduction of immortalizing genes or proteins, such as hTERT or oncogenes. The immortalization of cells may be reversible (*e.g*., using removable expression cassettes) or inducible (e.g., using inducible promoters).

Somatic cells as disclosed herein may be primary cells (non- immortalized cells), such as those freshly isolated from an animal, or may be derived from a cell line (immortalized cells). The cells may be maintained in cell culture following their isolation from a subject. It is disclosed that the cells are passaged once or more than once (*e.g*., between 2-5, 5-10, 10-20, 20-50, 50-100 times, or more) prior to their use in a method of the invention. It is disclosed that the cells will have been passaged no more than 1, 2, 5, 10, 20, or 50 times prior to their use in a method of the invention. They may be frozen, thawed, *etc.*

The somatic cells used or described herein may be native somatic cells, or engineered somatic cells, *i.e.,* somatic cells which have been genetically altered. Somatic cells as disclosed herein are typically mammalian cells, such as, for example, human cells, primate cells or mouse cells. They may be obtained by well-known methods and can be obtained from any organ or tissue containing live somatic cells, *e.g.,* blood, bone marrow, skin, lung, pancreas, liver, stomach, intestine, heart, reproductive organs, bladder, kidney, urethra and other urinary organs, *etc.*

Mammalian somatic cells useful in the present disclosure include, but are not limited to, Sertoli cells, endothelial cells, granulosa epithelial, neurons, pancreatic islet cells, epidermal cells, epithelial cells, hepatocytes, hair follicle cells, keratinocytes, hematopoietic cells, melanocytes, chondrocytes, lymphocytes (B and T lymphocytes), erythrocytes, macrophages, monocytes, mononuclear cells, cardiac muscle cells, and other muscle cells, *etc.*

It is also disclosed that cells are selected based on their expression of an endogenous marker known to be expressed only or primarily in a desired cell type or expression of an expression cassette under the control of a condition-responsive regulatory element. For example, vimentin is a fibroblast marker. Other useful markers include various keratins, cell adhesion molecules such as cadherins, fibronectin, CD molecules, *etc.* The population of somatic cells may have an average cell cycle time of between 18 and 96 hours, *e.g.,* between 24-48 hours, between 48-72 hours, *etc.* In some embodiments, at least 90%, 95%, 98%, 99%, or more of the cells would be expected to divide within a predetermined time such as 24, 48, 72, or 96 hours.

Methods described herein may be used to program one or more somatic cells, *e.g.,* colonies or populations of somatic cells into hepatocytes.

A population of cells as disclosed herein may be substantially uniform in that at least 90% of the cells display a phenotype or characteristic of interest. It is also disclosed that at least 95%, 96%, 97%, 98%, 99%, 99.5%, 99.8%, 99.9, 99.95% or more of the cells display a phenotype or characteristic of interest. It is also disclosed that the somatic cells have the capacity to divide, *i.e.,* the somatic cells are not post-mitotic.

Somatic cells may be partially or completely differentiated. Differentiation is the process by which a less specialized cell becomes a more specialized cell type. Cell differentiation can involve changes in the size, shape, polarity, metabolic activity, gene expression and/or responsiveness to signals of the cell. For example, hematopoietic stem cells differentiate to give rise to all the blood cell types including myeloid (monocytes and macrophages, neutrophils, basophils, eosinophils, erythrocytes, megakaryocytes/platelets, dendritic cells) and lymphoid lineages (T-cells, B-cells, NK-cells). During progression along the path of differentiation, the ultimate fate of a cell becomes more fixed. As described herein, both partially differentiated somatic cells and fully differentiated somatic cells can be programmed as described herein to produce desired cell types such as hepatocytes.

### III. Condition-responsive Regulatory Elements

Certain aspects of the invention provide methods and compositions for determination of biological response and/or pharmacologic effects on target tissue types in cell populations cultured *in vitro.* The cells contain a variety of expression cassettes comprising different condition-responsive regulatory elements controlling the expression of a selectable or screenable marker that reflects a status change in the cell, like a change in differentiation status, or a toxicologic or metabolic change, such as may be caused by a drug candidate that is present in the culture medium. The condition-responsive regulatory elements may be taken from a gene known to be upregulated when a tissue-specific, cell-specific, differentiation-specific, or molecular-pathway-specific response is activated or a particular toxicologic or other metabolic effect takes place in the cell. It controls transcription of a marker gene that provides an external signal that can be monitored as an indication of regulatory element activity. This system enables rapid high-throughput screening of a panel of culturing conditions for directed differentiation or a panel of test agents for potential toxicity and other metabolic effects on the cell.

"A condition-responsive regulatory element," as used herein, refers to a nucleotide sequence that regulates (*e.g*., up-regulates or down-regulates) transcription in response to a specific cellular condition, for example, a condition involving programming to a specific cell type or activation of a cell signaling pathway. For example, these sequences may be modular in nature, consisting of arrays of short (10- to 12-base pair) recognition elements that interact with specific transcription factors. Positive and negative regulatory elements that function only in specific cell types or in response to extracellular inducers have been identified and could be predicted by bioinfomratic analysis. A number of cases of inducible and tissue-specific gene expression involve the activation of preexisting transcription factors, rather than the synthesis of new proteins. This activation may involve covalent modification of the protein or an allosteric change in its structure.

### B. Condition-responsive regulatory elements

The exogenous expression cassettes in the set of cell lines may include any condition-responsive regulatory elements, especially promoters or enhancers specific for a selected tissue or cell lineage, or a selected signaling pathway, such as promoters of genes listed in Table 1. As indicated in the Table below, a tissue or lineage specific promoter can be specific to an organ, a generic cell type or a specific cell type. Thus a promoter, like Troponin T or alpha Myosin Heavy Chain, can be pan-cardiac, or expressing in all types of heart cells, while a promoter like sarcolipin can be used to specify atrial cells alone.

**Table 1: Examples of promoters for cell type-specific genes**

| | |
|---|---|
| Heart | troponin T promoter (known to be pan-cardiac) |
| | My12V promoter (known to be ventricular specific) |
| | Sarcolipin promoter (reported to be atrial specific) |
| Liver | Alpha-1-antitrypsin (AAT) promoter (endoderm) |
| | Cyp3A4 promoter (hepatocyte) |
| | HNF4a/FOXA2/HNF6 promoter (hepatocyte) |
| | HNF1b promoter (cholangiocytes) |
| Pancreas | PDX1 promoter (beta cells) |
| Intestine | IAP (Intestinal alkaline phosphatase) promoter (small intestine) |
| | Kruppel-like factor 4 (KLF4) promoter (large intestine) |
| Lung | Surfactant protein C (SP-C) promoter (lung epithelial type 2 cells) |
| | Surfactant protein B (SP-B) promoter (alveolar cells) |
| | Clara cell 10-Kd Protein promoter (airway Clara cells) |
| Endothelial | VE (vascular endothelial)-cadherin promoter (pan-endothelial) |
| Epithelial | Epithelial cell adhesion molecule promoter (pan-epithelial) |
| Blood | Vav promoter (early hematopoietic) |
| | Glycophorin A promoter (myeloid cells) |
| | Alpha-globin promoter (mature erythroid cells) |
| Neuron | BIII tubulin promoter (pan-neuron) |
| | Tyrosine hydroxylase (TH) promoter (dopaminergic) |
| | Glutamic Acid Decarboxylase (GAD67) promoter (GABAergic) |
| | Vesicular glutamate transporters (VGLUT1 or VGLUT2) promoter (glutamatergic) |
| | Glial fibrillary acidic protein (GFAP) promoter (astrocytes) |
| | O1 or 04 promoter (oligodendrocytes) |
| Adrenal gland | 24-dehydrocholesterol reductase promoter (cortical cell) |
| Prostate | Forkhead box A1 promoter (glandular cells) |
| Bladder | Uroplakin 3A promoter (urothelial cells) |
| Taste buds | Gustducin promoter (taste sensory cells) |
| Oral mucosa | Angiomotin like 2 promoter (squamous epithelial cells) |
| Tonsil | ST6 beta-galactosamide alpha-2,6-sialyltranferase 1 promoter (reaction center cells) |
| Kidney | Integrin alpha 8 promoter (glomeruli cells) |
| | Solute carrier family 12 (sodium/potassium/chloride transporters), member 1 promoter (the cells of the thick ascending limb of the loop of Henle in nephrons) |
| | Pollocalyxin-like promoter (podocytes in the Bowman's capsule) |
| Testes | Hydroxy-delta-5-steroid dehydrogenase, 3 beta- and steroid delta-isomerase 1 promoter (Leydig cells) |
| Salivary gland | Lactoperoxidase promoter (glandular cells) |
| Tooth | Ameloblastin (enamal matrix protein) promoter (ameloblast) |
| Endocrine system | Enolase 2 (gamma, neuronal) promoter (APUD cells in cerebral cortex, hippocampus, lateral ventrical, and cerebellum) |

Exemplary tissue-specific regulatory elements may include, but are not limited to one or more, even all of regulatory elements of genes specific for:
1) ORGANS: All organs in the human body. *E.g.,* kidney, heart, liver, pancreas, intestines *etc.*
2) ORGAN SUB-FRACTIONS: All organ subfractions in the human body. *E.g.,* Kidney medulla, kidney cortex, heart atria, heart ventricle, *etc.*

Example of cell-type specific categories include, but are not limited to:
1) CELL PROGENITORS: All relevant progenitor cell subtypes. *E.g.,* neural progenitors, hematopoietic progenitors, hepatocyte progenitors, cardiac progenitors, *etc.*
2) TERMINAL CELL TYPES: All terminal cell types in the human body. *E.g.,* Hepatocytes, cardiomyocytes, endothelial cells, neurons, *etc.*
3) TERMIMINAL CELL SUBTYPES: all terminal cell subtypes in the human body. E.g., Ventricular cardiomyocytes, atrial cardiomyocytes, nodal cardiomyocytes, arterial endothelial cells, venous endothelial cells, lymphatic endothelial cells, blood-brain barrier endothelial cells, dopaminergic neurons, cholinergic neurons, gabaergic neurons, motor neurons, *etc.*

Promoters or coding sequences for genes involved in various siganling pathways may be used in certain aspects of the present invention. Specifically, any promoter or transcription control element controlling a gene that is up- or down-regulated in response to a change in culture or cellular conditions (particularly the presence of a class of test drugs as well as up-regulation or down-regulation of a signaling pathway) may be suitable for use in certain aspects of this invention. Those signaling pathway genes are known in the art, for example, available via world wide web at invitrogen.com/site/us/en/home/Products-and-Services/Applications/Cell-and-Tissue-Analysis/Signaling-Pathways.html.

Exemplary signaling pathway genes may be involved in intracellular signaling pathways include, but are not limited to: tyrosine kinases, heterotrimeric G proteins, small GTPases, serine/threonine protein kinases, phosphatases, lipid kinases, hydrolases, chromatin regulation, MAPK signaling, Apoptosis/Autophagy, Translational Control, Cell Cycle/Checkpoint, DNA Damage, Jak/Stat Pathway, NF-κB Signaling, TGF-β/Smad signaling, lymphocyte signaling, angiogenesis, vesicle trafficking, cytoskeletal signaling, adhesion, glucose metabolism, Wnt/Hedgehog/Notch, stem cell/lineage markers, nuclear receptor, or protein folding and stability. Second messengers including: cyclic AMP (cAMP), cyclic GMP (cGMP), Phosphatidylinositol-triphosphate (PIP3), Diacylglycerol (DAG), Inositol-triphosphate (IP3). Adapter proteins including: EF hand domains of calmodulin, Pleckstrin homology domains of the kinase protein AKT or the like.

Examples of promoters having suitable characteristics also include the following:
Promoters for genes that respond to apoptosis, such as the PUMA gene. Drugs that trigger apoptosis may trigger promoters in this category. Other candidates are Gadd34, PUMA, GAHSP40, TRAIL-R2/DR5, c-fos, Gadd153, APAF-1, Gadd45, BTG2/PC3, Peg3/Pwl, Siah1a, S29 ribosomal protein, FasL/CD95L, tissue transglutaminase, GRP78, Nur77/NGFI-B, Cyclophilin D/CYPD, and P73.

Promoters for genes that respond to DNA damage, such as the p21, p21/WAF1, or Pig3 gene. Mutagens or teratogens may trigger promoters in this category.

Promoters for genes that respond to hyperplasia, such as the Ki-67 or Aurora A gene. Drugs that stimulate proliferation may trigger promoters in this category.

Promoters for genes that respond to oxidative stress. Heme oxygenase 1 (Hmox1), and superoxide dismutase (MnSOD) are upregulated with low oxygen levels; γ-glutamyl cysteinyl ligase (GCL), and Metallothionine I and II are upregulated by depletion of glutathione, or the presence of metal ions, respectively. Other candidates are IkB, ATF4, xanthine oxidase, COX2, iNOS, Ets-2, Cyclophilin A/CYPA, NQO1, and bNIP3.

Promoters for transcription factors that reflect changes in gene expression profiles upon initiation of any of these events, such as the PXR, CAR, aryl hydrocarbon receptor (AhR), or Nrf2 gene

Promoters for other hepatocyte markers that are upregulated in liver toxicity, such as Lrg-21, SOCS-2, SOCS-3, PAI-I, GBP28/adiponectin, α1-acid glycoprotein, ATF3, and Igfbp-3.

Promoters for genes that are responsive to receptors that act in the nucleus, exemplified by androgen, estrogen, and pPAG responsive gene. An example is the gene for prostate specific antigen (PSA).

Promoters for hepatocyte enzymes involved in drug metabolism that are also upregulated in the presence of substrate. Exemplary are cytochrome P450 genes, such as CYP3A4 and CYP1A1.

Promoter for drug transporter genes also upregulated by substrate, such as MDR1.

Promoters for genes that affect the contraction rate or the QT interval of the heart, such as calcium flux genes.

Promoters for genes controlling a product that is deficient in certain clinical conditions, and for which it may be useful to screen drugs that can regulate expression. Exemplary are genes that control hormone expression (e.g., insulin, or cortisol), and genes that control synthesis, release, metabolism, or reuptake of neurotransmitters (e.g., the serotonin transporter and tyrosine hydroxylase).

These and other promoters referred to in this disclosure can be cloned by amplification from a suitable genomic library using primers specific for the desired sequence, constructed using sequence data from such sources as GenBank.

In a particular example, tissue-specific transgene expression, especially for marker gene expression in hepatocytes derived from programming, is desirable as a way to identify derived hepatocytes. To increase both specificity and activity, the use of cis-acting regulatory elements has been contemplated. For example, a hepatocyte-specific promoter may be used, such as a promoter of albumin, α-1-antitrypsin (AAT), cytochrome p450 3A4 (CYP3A4), apolipoprotein A-I, or APOE.

In certain aspects, this also concerns enhancer sequences, *i.e.,* nucleic acid sequences that increase a promoter's activity and that have the potential to act in cis, and regardless of their orientation, even over relatively long distances (up to several kilobases away from the target promoter). However, enhancer function is not necessarily restricted to such long distances as they may also function in close proximity to a given promoter. For the liver, numerous approaches to incorporate such organ-specific regulatory sequences into retroviral, lentiviral, adenoviral and adeno-associated viral vectors or non-viral vectors (often in addition to house-keeping hepatocyte-specific cellular promoters) have been reported so far (Ferry *et al.,* 1998; Ghosh *et al.,* 2000; Miao *et al.,* 2000; Follenzi *et al.,* 2002).

Several enhancer sequences for liver-specific genes have been documented. WO2009130208 describes several liver-specific regulatory enhancer sequences. WO95/011308 describes a gene therapy vector comprising a hepatocyte-specific control region (HCR) enhancer linked to a promoter and a transgene. The human apolipoprotein E-Hepatocyte Control Region (ApoE-HCR) is a locus control region (LCR) for liver-specific expression of the apolipoprotein E (ApoE) gene. The ApoE-HCR is located in the ApoE/CI/CII locus, has a total length of 771 bp and is important in expression of the genes ApoE and ApoC-1 in the liver (Simonet *et al.,* 1993). In WO01/098482, the combination of this specific ApoE enhancer sequence or a truncated version thereof with hepatic promoters is suggested. It was shown that vector constructs combining the (non-truncated) ApoE-HCR enhancer with a human alpha-antitrypsin (AAT) promoter were able to produce the highest level of therapeutic protein *in vivo* (Miao *et al.,* 2000) and may confer sustained expression when used in conjunction with a heterologous transgene (Miao *et al.,* 2001).

Other chimeric liver-specific constructs have also been proposed in the literature, *e.g.,* with the AAT promoter and the albumin or hepatitis B enhancers (Kramer *et al.,* 2003), or the alcohol dehydrogenase 6 (ADH6) basal promoter linked to two tandem copies of the apolipoprotein E enhancer element (Gehrke *et al.,* 2003). The authors of the latter publication stress the importance of the relatively small size (1068 bp) of this enhancer-promoter combination.

### C. Promoter identification and characterization

Currently a collection of over 17,000 human promoters (available from SwitchGear Genomics) could be integrated into exogenous expression cassettes of the present invention.

Regulation is the complex orchestration of events starting with an extracellular signal such as a hormone and leading to an increase or decrease in the activity of one or more proteins. Bioinformatics techniques could be applied to explore various steps in this process. For example, promoter analysis involves the identification and study of sequence motifs in the DNA surrounding the coding region of a gene. These motifs influence the extent to which that region is transcribed into mRNA.

Regulation of expression is determined to a large extent by the promoter sequences of the individual genes (and/or enhancers). The complete sequence of the human genome now provides the molecular basis for the identification of many regulatory regions. For example, promoter sequences for specific cDNAs can be obtained reliably from genomic sequences by exon mapping. In the many cases in which cDNAs are 5'-incomplete, high quality promoter prediction tools can be used to locate promoters directly in the genomic sequence.

Significant improvements in promoter prediction have been made within the last few years. PromoterScan (Prestridge, 1995) has been viewed as one of the first promoter prediction algorithms with acceptably high specificity. Recently, PromoterInspector (Scherf *et al.,* 2000) and Dragon Promoter Finder (Bajic *et al.,* 2002) made further progress in specificity and sensitivity of promoter prediction algorithms. PromoterScan identifies promoters using a TATA box positional weight matrix combined with the density of specific transcription factor binding sites. The algorithm has been demonstrated to be of comparatively high specificity but low sensitivity.

An effective promoter identification algorithm, which is called PromoterExplorer, has been proposed recently by Xie *et al.* (2006). In this approach, various features such as local distribution of pentamers, positional CpG island features and digitized DNA sequence are combined to build a high-dimensional input vector and then a cascade AbaBoost algorithm is used both to perform feature selection and classifier training.

Expression data can also be used to infer gene regulation: one might compare microarray data from a wide variety of states of an organism to form hypotheses about the genes involved in each state. In a single-cell organism, one might compare stages of the cell cycle, along with various stress conditions (heat shock, starvation, *etc*.). One can then apply clustering algorithms to that expression data to determine which genes are co-expressed. For example, the upstream regions (promoters) of co-expressed genes can be searched for over-represented regulatory elements.

### IV. Expression cassettes

The present invention involve use of exogenous expression cassettes including a condition-responsive regulatory element that regulates the expression of a selectable or screenable marker that provides an external signal for monitoring the regulatory element activity. It is disclosed that the expression cassettes can convey a polycistronic message for efficient co-expression of multiple genes.

### B. Polycistronic message

In the context of the present invention it is disclosed that the fexibility and efficient expression from this polycistronic system underlie its advantages and establish it as a useful tool to provide engineered cells. The various permutations of this system include but are not
to: 1) including at least two markers in a polycistronic transcript, such as both a selectable and a screenable marker, two different selectable markers or two different screenable markers, 2) including one or more markers in combination with a non-marker coding sequence such as a drug metabolism enzyme gene or programming gene, or 3) creating a cassette with at least three coding sequences, for example, using at least two IRES sites or 2A peptides.

### 2. Protease cleavage site or self-cleaving peptide for polycistronic expression

It is disclosed that the genes encoding markers or other proteins may be connected to one another by a sequence (there may be more than one) coding for a protease cleavage site (*i.e.,* a sequence comprising the recognition site of a protease) or at least one self-cleaving peptide.

It is also disclosed that the protease(s) capable of cleaving the cleavage sites encoded by the sequence(s) connecting the genes constituting the polycistronic message is/are encoded by the polynucleotide as disclosed herein. More preferably, the gene(s) encoding the protease(s) is/are part of at least the polycistronic meassage.

Suitable protease cleavages sites and self-cleaving peptides are known to the skilled person (see, *e.g.,* in Ryan *et al.,* 1997; Scymczak *et al.,* 2004). Preferred examples of protease cleavage sites are the cleavage sites of potyvirus NIa proteases (*e.g.,* tobacco *etc*h virus protease), potyvirus HC proteases, potyvirus P1 (P35) proteases, byovirus Nla proteases, byovirus RNA-2- encoded proteases, aphthovirus L proteases, enterovirus 2A proteases, rhinovirus 2A proteases, picorna 3C proteases, comovirus 24K proteases, nepovirus 24K proteases, RTSV (rice tungro spherical virus) 3Ciike protease, PY\IF (parsnip yellow fleck virus) 3C-like protease, thrombin, factor Xa and enterokinase.

Due to its high cleavage stringency, TEV (tobacco etch virus) protease cleavage sites are particularly preferred. Thus, the genes of the polygenes according to the present invention are preferably connected by a stretch of nucleotides comprising a nucleotide sequence encoding an amino acid sequence of the general, form E)(XYXQ(G/S) wherein X represents any amino acid (cleavage by TEV occurs between Q and G or Q. and S). Most preferred are linker nucleotide sequences coding for ENLYFQG and ENLYFQS, respectively.

Preferred self-cleaving peptides (also called "cis-acting hydrolytic elements", CHYSEL; see deFelipe (2002)) are derived from potyvirus and cardiovirus 2A peptides. Especially preferred self-cleaving peptides are selected from 2A peptides derived from FMDV (foot-and-mouth disease virus), equine rhinitis A virus, Thoseà asigna virus and porcine teschovirus.

The polypeptides encoded by the nucleotide sequences constituting the polycistronic meassage as disclosed herein may be the same or different. Thus, each polygene present in the constructs of the invention may contain one or more copy of each nucleotide sequence encoding a protein of interest.

### 3. IRES

There is disclosed that the use of internal ribosome entry sites (IRES) elements are used to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent Nos. 5,925,565 and 5,935,819).

Most eukaryotic and viral messages initiate translation by a mechanism involving recognition of a 7-methylguanosine cap at the 5' end of the mRNA. In a few cases, however, translation occurs via a cap-independent mechanism in which an internal ribosome entry site (IRES) positioned 3' downstream of the gene translated from the cap region of the mRNA is recognized by the ribosome, allowing translation of a second coding region from the transcript. Therefore, IRES elements are able to bypass the ribosome scanning model of 5' methylated Cap dependent translation and begin translation at internal sites (Pelletier and Sonenberg, 1988).

This is particularly important in the context of the present disclosure as an IRES sequence allows simultaneous expression of multiple proteins from a single genetic locus. IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent Nos. 5,925,565 and 5,935,819).

There are also disclosed coding sequences for both a desired recombinant product and a selectable or screenable marker within the same polycistronic transcript. Successful transformation events are marked by both expression of the desired reprogramming factors or drug-responsive genes and the easily detectable selectable or screenable markers, facilitating selection of successfully transfected cells.

IRES elements from two members of the picornavirus family (polio and encephalomyocarditis) have been described (Pelletier and Sonenberg, 1988), as well an IRES from a mammalian message (Macejak and Sarnow, 1991). Certain examples include those IRES elements from poliovirus Type I, the 5'UTR of encephalomyocarditis virus (EMV), of "Thelier's murine encephalomyelitis virus (TMEV), of "foot and mouth disease virus" (FMDV), of "bovine enterovirus (BEV), of "coxsackie B virus" (CBV), or of "human rhinovirus" (HRV), or the "human immunoglobulin heavy chain binding protein" (BIP) 5'UTR, the Drosophila antennapediae 5'UTR or the Drosophila ultrabithorax 5'UTR, or genetic hybrids or fragments from the above-listed sequences. IRES sequences are described in Kim *et al.* (1992) and McBratney *et al.* (1993).

It is disclosed that a polycistronic transcript may be used by employing one or more internal ribosome entry sites (IRESs). Exemplary IRES may be an encephalomyocarditis virus IRES, a picornavirus IRES, a foot-and-mouth disease virus IRES, a hepatitis A virus IRES, a hepatitis C virus IRES, a human rhinovirus IRES, a poliovirus IRES, a swine vesicular disease virus IRES, a turnip mosaic potyvirus IRES, a human fibroblast growth factor 2 mRNA IRES, a pestivirus IRES, a Leishmania RNA virus IRES, a Moloney murine leukemia virus IRES a human rhinovirus 14 IRES, an aphthovirus IRES, a human immunoglobulin heavy chain binding protein mRNA IRES, a Drosophila Antennapedia mRNA IRES, a human fibroblast growth factor 2 mRNA IRES, a hepatitis G virus IRES, a tobamovirus IRES, a vascular endothelial growth factor mRNA IRES, a Coxsackie B group virus IRES, a c-myc protooncogene mRNA IRES, a human MYT2 mRNA IRES, a human parechovirus type 1 virus IRES, a human parechovirus type 2 virus IRES, a eukaryotic initiation factor 4GI mRNA IRES, a Plautia stali intestine virus IRES, a Theiler's murine encephalomyelitis virus IRES, a bovine enterovirus IRES, a connexin 43 mRNA IRES, a homeodomain protein Gtx mRNA IRES, an AML1 transcription factor mRNA IRES, an NF-kappa B repressing factor mRNA IRES, an X-linked inhibitor of apoptosis mRNA IRES, a cricket paralysis virus RNA IRES, a p58 (PITSLRE) protein kinase mRNA IRES, an ornithine decarboxylase mRNA IRES, a connexin-32 mRNA IRES, a bovine viral diarrhea virus IRES, an insulin-like growth factor I receptor mRNA IRES, a human immunodeficiency virus type 1 gag gene IRES, a classical swine fever virus IRES, a Kaposi's sarcoma-associated herpes virus IRES, a short IRES selected from a library of random oligonucleotides, a Jembrana disease virus IRES, an apoptotic protease-activating factor 1 mRNA IRES, a Rhopalosiphum padi virus IRES, a cationic amino acid transporter mRNA IRES, a human insulin-like growth factor II leader 2 mRNA IRES, a giardiavirus IRES, a Smad5 mRNA IRES, a porcine teschovirus-1 talfan IRES, a Drosophila Hairless mRNA IRES, an hSNM1 mRNA IRES, a Cbfa1/Runx2 mRNA IRES, an Epstein-Barr virus IRES, a hibiscus chlorotic ringspot virus IRES, a rat pituitary vasopressin V1b receptor mRNA IRES, a human hsp70 mRNA IRES, or a variant thereof.

### C. Selection and Screenable Markers

In certain embodiments of the invention, cells containing a nucleic acid construct of the present invention may be identified *in vitro* or *in vivo* by including a marker in the expression cassette. Such markers would confer an identifiable change to the cell permitting easy identification of cells containing the expression cassette. Generally, a selection marker is one that confers a property that allows for selection. A positive selection marker is one in which the presence of the marker allows for its selection, while a negative selection marker is one in which its presence prevents its selection. An example of a positive selection marker is a drug resistance marker.

Usually the inclusion of a drug selection marker aids in the cloning and identification of transformants, for example, genes that confer resistance to neomycin, puromycin, blastocidin, geneticin, hygromycin, DHFR, GPT, zeocin and histidinol are useful selection markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes as negative selection markers such as herpes simplex virus thymidine kinase (*tk*) or chloramphenicol acetyltransferase (CAT) may be utilized. One of skill in the art would also know how to employ immunologic markers, possibly in conjunction with FACS analysis. The marker used is not believed to be important, so long as it is capable of being expressed simultaneously with the nucleic acid encoding a gene product. Further examples of selection and screenable markers are well known to one of skill in the art.

Certain embodiments of the present invention utilize screenable reporter genes to indicate specific property of cells, for example, differentiation along a defined cell lineage by activating a condition-responsive regulatory element which controls the reporter marker gene expression.

Examples of such reporters include genes encoding cell surface proteins (*e.g*., CD4, HA epitope), fluorescent proteins, antigenic determinants and enzymes *(e.g.,* β-galactosidase or a nitroreductase). The vector containing cells may be isolated, *e.g.,* by FACS using fluorescently-tagged antibodies to the cell surface protein or substrates that can be converted to fluorescent products by a vector encoded enzyme. In certain aspects cell-permeable dyes can be used to identify cells expressing a resporter. For example, expression of a NFAT nitroreductase gene can be detected by using a cell permeable pro-fluorogenic substrate such as CytoCy5S (see, *e.g.,* U.S. Patent Nos. 5,633,158, 5,780,585, 5,977,065 and EP Patent No. EP 1252520).

In specific embodiments, the reporter gene is a fluorescent protein. A broad range of fluorescent protein genetic variants have been developed that feature fluorescence emission spectral profiles spanning almost the entire visible light spectrum (see Table 2 for non-limiting examples). Mutagenesis efforts in the original Aequorea victoria jellyfish green fluorescent protein have resulted in new fluorescent probes that range in color from blue to yellow, and are some of the most widely used *in vivo* reporter molecules in biological research. Longer wavelength fluorescent proteins, emitting in the orange and red spectral regions, have been developed from the marine anemone, Discosoma striata, and reef corals belonging to the class Anthozoa. Still other species have been mined to produce similar proteins having cyan, green, yellow, orange, and deep red fluorescence emission. Developmental research efforts are ongoing to improve the brightness and stability of fluorescent proteins, thus improving their overall usefulness.

**Table 2: Fluorescent Protein Properties**

| Protein (Acronym) | Excitation Maximum (nm) | Emission Maximum (nm) | Molar Extinction Coefficient | Quantum Yield | *in vivo* Structure | Relative Brightness (% of EGFP) |
|---|---|---|---|---|---|---|
| GFP (wt) | 395/475 | 509 | 21,000 | 0.77 | Monomer* | 48 |

| Green Fluorescent Proteins | | | | | | |
|---|---|---|---|---|---|---|
| EGFP | 484 | 507 | 56,000 | 0.60 | Monomer* | 100 |
| AcGFP | 480 | 505 | 50,000 | 0.55 | Monomer* | 82 |
| TurboGFP | 482 | 502 | 70,000 | 0.53 | Monomer* | 110 |
| Emerald | 487 | 509 | 57,500 | 0.68 | Monomer* | 116 |
| Azami Green | 492 | 505 | 55,000 | 0.74 | Monomer | 121 |
| ZsGreen | 493 | 505 | 43,000 | 0.91 | Tetramer | 117 |

| Blue Fluorescent Proteins | | | | | | |
|---|---|---|---|---|---|---|
| EBFP | 383 | 445 | 29,000 | 0.31 | Monomer* | 27 |
| Sapphire | 399 | 511 | 29,000 | 0.64 | Monomer* | 55 |
| T-Sapphire | 399 | 511 | 44,000 | 0.60 | Monomer* | 79 |

| Cyan Fluorescent Proteins | | | | | | |
|---|---|---|---|---|---|---|
| ECFP | 439 | 476 | 32,500 | 0.40 | Monomer* | 39 |
| mCFP | 433 | 475 | 32,500 | 0.40 | Monomer | 39 |
| Cerulean | 433 | 475 | 43,000 | 0.62 | Monomer* | 79 |
| CyPet | 435 | 477 | 35,000 | 0.51 | Monomer* | 53 |
| AmCyan1 | 458 | 489 | 44,000 | 0.24 | Tetramer | 31 |
| Midori-Ishi Cyan | 472 | 495 | 27,300 | 0.90 | Dimer | 73 |
| mTFP1 (Teal) | 462 | 492 | 64,000 | 0.85 | Monomer | 162 |

| Yellow Fluorescent Proteins | | | | | | |
|---|---|---|---|---|---|---|
| EYFP | 514 | 527 | 83,400 | 0.61 | Monomer* | 151 |
| Topaz | 514 | 527 | 94,500 | 0.60 | Monomer* | 169 |
| Venus | 515 | 528 | 92,200 | 0.57 | Monomer* | 156 |
| mCitrine | 516 | 529 | 77,000 | 0.76 | Monomer | 174 |
| YPet | 517 | 530 | 104,000 | 0.77 | Monomer* | 238 |
| PhiYFP | 525 | 537 | 124,000 | 0.39 | Monomer* | 144 |
| ZsYellow1 | 529 | 539 | 20,200 | 0.42 | Tetramer | 25 |
| mBanana | 540 | 553 | 6,000 | 0.7 | Monomer | 13 |

| Orange and Red Fluorescent Proteins | | | | | | |
|---|---|---|---|---|---|---|
| Kusabira Orange | 548 | 559 | 51,600 | 0.60 | Monomer | 92 |
| mOrange | 548 | 562 | 71,000 | 0.69 | Monomer | 146 |
| dTomato | 554 | 581 | 69,000 | 0.69 | Dimer | 142 |
| dTomato-Tandem | 554 | 581 | 138,000 | 0.69 | Monomer | 283 |
| DsRed | 558 | 583 | 75,000 | 0.79 | Tetramer | 176 |
| DsRed2 | 563 | 582 | 43,800 | 0.55 | Tetramer | 72 |
| DsRed-Express (T1) | 555 | 584 | 38,000 | 0.51 | Tetramer | 58 |
| DsRed-Monomer | 556 | 586 | 35,000 | 0.10 | Monomer | 10 |
| mTangerine | 568 | 585 | 38,000 | 0.30 | Monomer | 34 |
| mStrawberry | 574 | 596 | 90,000 | 0.29 | Monomer | 78 |
| AsRed2 | 576 | 592 | 56,200 | 0.05 | Tetramer | 8 |
| mRFP1 | 584 | 607 | 50,000 | 0.25 | Monomer | 37 |
| JRed | 584 | 610 | 44,000 | 0.20 | Dimer | 26 |
| mCherry | 587 | 610 | 72,000 | 0.22 | Monomer | 47 |
| HcRed1 | 588 | 618 | 20,000 | 0.015 | Dimer | 1 |
| mRaspberry | 598 | 625 | 86,000 | 0.15 | Monomer | 38 |
| HcRed-Tandem | 590 | 637 | 160,000 | 0.04 | Monomer | 19 |
| mPlum | 590 | 649 | 41,000 | 0.10 | Monomer | 12 |
| AQ143 | 595 | 655 | 90,000 | 0.04 | Tetramer | 11 |
| | | | | | * Weak Dimer | |

### D. Allelic Variants

As disclosed herein the set of cell lines or the expression cassettes may further comprise additional coding sequence for drug metabolizing enzyme or drug targets and variants thereof. One benefit of using pluripotent stem cells is the ability to make cells that are identical in all respects, except that they have a particular variation in the gene encoding a drug metabolizing enzyme or drug target of particular interest. This is relevant in the context of drug screening, because there are some naturally occurring allelic variants that affect an individual's ability to respond to or metabolize drugs of a particular class. Because the cells are otherwise the same, the user can determine the effect of the compound being screened in an allotype specific manner. See published U.S. patent application 2003/0003573.

Examples of drug metabolizing enzymes having known allelic variants of consequence are described by Wolf *et al.,* 2000; Wolf *et al.,* 1999; and Webber, 1997.

**Table 3: Naturally Occurring Allotype Variants of Drug Metabolizing Enzymes**

| Enzyme | Variant phenotype | Frequency | Total No. of Drugs | Exemplary Substrates |
|---|---|---|---|---|
| CYP2D6 | poor metabolizer | White 6%; African American 2%; Oriental 1% | >100 | codeine, nortryptiline, dextromethorphan |
| | ultra-rapid metabolizer | Ethiopian 20%; Spanish 7%; Scandinavian 1.5% | | |
| CYP2C9 | reduced activity | | >60 | tolbutamide, diazepam, ibuprophen, warfarin |
| CYP2C19 | poor metabolizer | Oriental 23%; White 4% | >50 | mephenytoin, omeprazole, proguanil, citalopram |
| N-acetyl transferase | poor metabolizer | White 60%; African American 60%; Oriental 20%; Inuit 5% | >15 | isoniazid, procainamaide, sulphonamides, hydralazines |
| Thiopurine methyltransferase | poor metabolizer | low in all populations | <10 | 6-mercaptopurine, 6-thioguanine, azathioprine |

Another enzyme with known variants is CYP3A4, which plays a role in deactivating testosterone, and which is implicated in susceptibility to prostate cancer (Paris *et al.,* 1999).

To put into effect this, pluripotent stem cells may be divided into two or more separate subsets. One or more of the cell lines may be genetically altered to introduce a variant of the gene for the drug metabolizing enzyme or drug target (before or after introduction of the exogenous expression cassette). The gene can be introduced by random transduction, but more typically the variant is substituted for the native gene by homologous recombination. This both silences the endogenous gene, and places the variant under control of condition-responsive regulatory elements, for example, cell-specific or inducible promoters. Alternatively, if a naturally occurring variant is known to differ from the usual gene by a point mutation, the endogenous gene can be mutated so as to confer the same phenotype while introducing a condition-responsive transcription regulatory element for regulating the variant expression. The user has the option of altering the opposite allele to express the same variant, or inactivating it, for example, by homologous recombination.

The cells could then differentiated and used for drug screening as described in the sections that follow.

### V. Delivery of gene or gene products

As disclosed herein, vectors for delivery of nucleic acids encoding exogenous condition-responsive expression cassettes could be constructed to express these factors in cells. In a particular aspect, the following systems and methods may be used in delivery of expression cassette for identification of desired cell types. In particular, a set of stem cell lines may comprise a set of different expression cassettes, each expression cassette under the control of a different condition-responsive regulatory element for expression in responsive to a defined condition, such as differentiation to a defined cell lineage.

### B. Homologous recombination

As disclosed herein, the exogenous expression cassettes such as condition-responsive expression cassettes or reprogramming cassettes may be introduced into cells in a specific manner, for example, via homologous recombination. Current approaches to express genes in stem cells have involved the use of viral vectors or transgenes that integrate randomly in the genome. These approaches have not been successful due in part because the randomly integrated vectors can activate or suppress endogenous gene expression, and/or the silencing of transgene expression. The problems associated with random integration could be partially overcome by homologous recombination to a specific locus in the target genome, *e.g*., a Rosa26 locus. The Rosa26 locus is easily accessible and amenable to homologous recombination. Transgenes targeted by homologous recombination to the Rosa26 locus are stably and efficiently expressed in the undifferentiated cells as well as the differentiated cell types generated from stem cells such as mouse or human pluripotent stem cells.

Homologous recombination (HR), also known as general recombination, is a type of genetic recombination used in all forms of life in which nucleotide sequences are exchanged between two similar or identical strands of DNA. The technique has been the standard method for genome engineering in mammalian cells since the mid 1980s. The process involves several steps of physical breaking and the eventual rejoining of DNA. This process is most widely used to repair potentially lethal double-strand breaks in DNA. In addition, homologous recombination produces new combinations of DNA sequences during meiosis, the process by which eukaryotes make germ cells like sperm and ova. These new combinations of DNA represent genetic variation in offspring which allow populations to evolutionarily adapt to changing environmental conditions over time. Homologous recombination is also used in horizontal gene transfer to exchange genetic material between different strains and species of bacteria and viruses. Homologous recombination is also used as a technique in molecular biology for introducing genetic changes into target organisms.

Homologous recombination can be used as targeted genome modification. The efficiency of standard HR in mammalian cells is only 10⁻⁶ to 10⁻⁹ of cells treated (Capecchi, 1990). The use of meganucleases, or homing endonucleases, such as I-SceI have been used to increase the efficiency of HR. Both natural meganucleases as well as engineered meganucleases with modified targeting specificities have been utilized to increase HR efficiency (Pingoud and Silva, 2007; Chevalier et al., 2002). nother path toward increasing the efficiency of HR has been to engineer chimeric endonucleases with programmable DNA specificity domains (Silva et al., 2011). Zinc-finger nucleases (ZFN) are one example of such a chimeric molecule in which Zinc-finger DNA binding domains are fused with the catalytic domain of a Type IIS restriction endonuclease such as FokI (as reviewed in Durai *et al.,* 2005; PCT/US2004/030606). Another class of such specificity molecules includes Transcription Activator Like Effector (TALE) DNA binding domains fused to the catalytic domain of a Type IIS restriction endonuclease such as FokI (Miller et al., 2011: PCT/IB2010/000154).

### C. Nuclei acid delivery systems

One of skill in the art would be well equipped to construct a vector through standard recombinant techniques (see, for example, Sambrook *et al.,* 2001 and Ausubel *et al.,* 1996). Vectors include but are not limited to, plasmids, cosmids, viruses (bacteriophage, animal viruses, and plant viruses), and artificial chromosomes *(e.g.,* YACs), such as retroviral vectors *(e.g.,* derived from Moloney murine leukemia virus vectors (MoMLV), MSCV, SFFV, MPSV, SNV *etc*), lentiviral vectors *(e.g.,* derived from HIV-1, HIV-2, SIV, BIV, FIV *etc.*), adenoviral (Ad) vectors including replication competent, replication deficient and gutless forms thereof, adeno-associated viral (AAV) vectors, simian virus 40 (SV-40) vectors, bovine papilloma virus vectors, Epstein-Barr virus, herpes virus vectors, vaccinia virus vectors, Harvey murine sarcoma virus vectors, murine mammary tumor virus vectors, Rous sarcoma virus vectors.

### 2. Episomal Vectors

The use of plasmid- or liposome-based extra-chromosomal (*i.e.,* episomal) vectors is also disclosed in the context of the present invention for example, for reprogramming of somatic cells. Such episomal vectors may include, *e.g.,* oriP-based vectors, and/or vectors encoding a derivative of EBNA-1. These vectors may permit large fragments of DNA to be introduced to a cell and maintained extra-chromosomally, replicated once per cell cycle, partitioned to daughter cells efficiently, and elicit substantially no immune response.

In particular, EBNA-1, the only viral protein required for the replication of the oriP-based expression vector, does not elicit a cellular immune response because it has developed an efficient mechanism to bypass the processing required for presentation of its antigens on MHC class I molecules (Levitskaya *et al.,* 1997). Further, EBNA-1 can act in *trans* to enhance expression of the cloned gene, inducing expression of a cloned gene up to 100-fold in some cell lines (Langle-Rouault *et al.,* 1998; Evans *et al.,* 1997). Finally, the manufacture of such oriP-based expression vectors is inexpensive.

Other extra-chromosomal vectors include other lymphotrophic herpes virus-based vectors. Lymphotrophic herpes virus is a herpes virus that replicates in a lymphoblast (e.g., a human B lymphoblast) and becomes a plasmid for a part of its natural life-cycle. Herpes simplex virus (HSV) is not a "lymphotrophic" herpes virus. Exemplary lymphotrophic herpes viruses include, but are not limited to EBV, Kaposi's sarcoma herpes virus (KSHV); Herpes virus saimiri (HS) and Marek's disease virus (MDV). Also other sources of episome-base vectors are contemplated, such as yeast ARS, adenovirus, SV40, or BPV.

One of skill in the art would be well equipped to construct a vector through standard recombinant techniques (see, for example, Maniatis *et al.,* 1988 and Ausubel *et al.,* 1994).

Vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide.

Such components also might include markers, such as detectable and/or selection markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities. A large variety of such vectors are known in the art and are generally available. When a vector is maintained in a host cell, the vector can either be stably replicated by the cells during mitosis as an autonomous structure, incorporated within the genome of the host cell, or maintained in the host cell's nucleus or cytoplasm.

### 3. Transposon-based system

According to a particular embodiment the introduction of nucleic acids may use a transposon - transposase system. The used transposon - transposase system could be the well known Sleeping Beauty, the Frog Prince transposon - transposase system (for the description of the latter see *e.g*., EP1507865), or the TTAA-specific transposon piggyBac system.

Transposons are sequences of DNA that can move around to different positions within the genome of a single cell, a process called transposition. In the process, they can cause mutations and change the amount of DNA in the genome. Transposons were also once called jumping genes, and are examples of mobile genetic elements.

There are a variety of mobile genetic elements, and they can be grouped based on their mechanism of transposition. Class I mobile genetic elements, or retrotransposons, copy themselves by first being transcribed to RNA, then reverse transcribed back to DNA by reverse transcriptase, and then being inserted at another position in the genome. Class II mobile genetic elements move directly from one position to another using a transposase to "cut and paste" them within the genome.

### 4. Viral Vectors

In generating recombinant viral vectors, non-essential genes are typically replaced with a gene or coding sequence for a heterologous (or non-native) protein. Viral vectors are a kind of expression construct that utilizes viral sequences to introduce nucleic acid and possibly proteins into a cell. The ability of certain viruses to infect cells or enter cells *via* receptor-mediated endocytosis, and to integrate into host cell genome and express viral genes stably and efficiently have made them attractive candidates for the transfer of foreign nucleic acids into cells (e.g., mammalian cells). Non-limiting examples of virus vectors that may be used to deliver a nucleic acid of certain aspects of the present invention are described below.

Retroviruses have promise as gene delivery vectors due to their ability to integrate their genes into the host genome, transferring a large amount of foreign genetic material, infecting a broad spectrum of species and cell types and of being packaged in special cell-lines (Miller, 1992).

In order to construct a retroviral vector, a nucleic acid is inserted into the viral genome in the place of certain viral sequences to produce a virus that is replication-defective. In order to produce virions, a packaging cell line containing the gag, pol, and env genes but without the LTR and packaging components is constructed (Mann *et al.,* 1983). When a recombinant plasmid containing a cDNA, together with the retroviral LTR and packaging sequences is introduced into a special cell line (e.g., by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann *et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al.,* 1975).

Lentiviruses are complex retroviruses, which, in addition to the common retroviral genes *gag, pol,* and *env,* contain other genes with regulatory or structural function. Lentiviral vectors are well known in the art (see, for example, Naldini *et al.,* 1996; Zufferey *et al.,* 1997; Blomer *et al.,* 1997; U.S. Patents 6,013,516 and 5,994,136).

Recombinant lentiviral vectors are capable of infecting non-dividing cells and can be used for both *in vivo* and *ex vivo* gene transfer and expression of nucleic acid sequences. For example, recombinant lentivirus capable of infecting a non-dividing cell wherein a suitable host cell is transfected with two or more vectors carrying the packaging functions, namely gag, pol and env, as well as rev and tat is described in U.S. Patent 5,994,136.

### D. Nucleic acid Delivery

Introduction of a nucleic acid, such as DNA or RNA, into cells to be programmed with the current invention may use any suitable methods for nucleic acid delivery for transformation of a cell., as described herein or as would be known to one of ordinary skill in the art. Such methods include, but are not limited to, direct delivery of DNA such as by ex vivo transfection (Wilson *et al.,* 1989, Nabel *et al,* 1989), by injection (U.S. Patent Nos. 5,994,624, 5,981,274, 5,945,100, 5,780,448, 5,736,524, 5,702,932, 5,656,610, 5,589,466 and 5,580,859), including microinjection (Harland and Weintraub, 1985; U.S. Patent No. 5,789,215, incorporated herein by reference); by electroporation (U.S. Patent No. 5,384,253); Tur-Kaspa *et al.,* 1986; Potter *et al.,* 1984); by calcium phosphate precipitation (Graham and Van Der Eb, 1973; Chen and Okayama, 1987; Rippe *et al.,* 1990); by using DEAE-dextran followed by polyethylene glycol (Gopal, 1985); by direct sonic loading (Fechheimer *et al.,* 1987); by liposome mediated transfection (Nicolau and Sene, 1982; Fraley *et al.,* 1979; Nicolau *et al.,* 1987; Wong *et al.,* 1980; Kaneda *et al.,* 1989; Kato *et al.,* 1991) and receptor-mediated transfection (Wu and Wu, 1987; Wu and Wu, 1988); by microprojectile bombardment (PCT Application Nos. WO 94/09699 and 95/06128; U.S. Patent Nos. 5,610,042; 5,322,783 5,563,055, 5,550,318, 5,538,877 and 5,538,880);
by agitation with silicon carbide fibers (Kaeppler *et al.,* 1990; U.S. Patent Nos. 5,302,523 and 5,464,765); by *Agrobacterium*-mediated transformation (U.S. Patent Nos. 5,591,616 and 5,563,055);
by desiccation/inhibition-mediated DNA uptake (Potrykus *et al.,* 1985), and any combination of such methods. Through the application of techniques such as these, organelle(s), cell(s), tissue(s) or organism(s) may be stably or transiently transformed.

### 2. Liposome-Mediated Transfection

As disclosed herein, a nucleic acid may be entrapped in a lipid complex such as, for example, a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, 1991). Also contemplated is an nucleic acid complexed with Lipofectamine (Gibco BRL) or Superfect (Qiagen). The amount of liposomes used may vary upon the nature of the liposome as well as the cell used, for example, about 5 to about 20 µg vector DNA per 1 to 10 million of cells may be contemplated.

Liposome-mediated nucleic acid delivery and expression of foreign DNA *in vitro* has been very successful (Nicolau and Sene, 1982; Fraley *et al.,* 1979; Nicolau *et al.,* 1987). The feasibility of liposome-mediated delivery and expression of foreign DNA in cultured chick embryo, HeLa and hepatoma cells has also been demonstrated (Wong *et al.,* 1980).

As disclosed herein , a liposome may be complexed with a hemagglutinating virus (HVJ). This has been shown to facilitate fusion with the cell membrane and promote cell entry of liposome-encapsulated DNA (Kaneda *et al.,* 1989). In other embodiments, a liposome may be complexed or employed in conjunction with nuclear non-histone chromosomal proteins (HMG-1) (Kato *et al.,* 1991). It is also disclosed that a liposome may be complexed or employed in conjunction with both HVJ and HMG-1. It is also disclosed that a delivery vehicle may comprise a ligand and a liposome.

### 3. Electroporation

As disclosed herein , a nucleic acid is introduced into an organelle, a cell, a tissue or an organism *via* electroporation. Electroporation involves the exposure of a suspension of cells and DNA to a high-voltage electric discharge. Recipient cells can be made more susceptible to transformation by mechanical wounding. Also the amount of vectors used may vary upon the nature of the cells used, for example, about 5 to about 20 µg vector DNA per 1 to 10 million of cells may be contemplated.

Transfection of eukaryotic cells using electroporation has been quite successful. Mouse pre-B lymphocytes have been transfected with human kappa-immunoglobulin genes (Potter *et al.,* 1984), and rat hepatocytes have been transfected with the chloramphenicol acetyltransferase gene (Tur-Kaspa *et al.,* 1986) in this manner.

### 4. Calcium Phosphate

As disclosed herein , a nucleic acid is introduced to the cells using calcium phosphate precipitation. Human KB cells have been transfected with adenovirus 5 DNA (Graham and Van Der Eb, 1973) using this technique. Also in this manner, mouse L(A9), mouse C127, CHO, CV-1, BHK, NIH3T3 and HeLa cells were transfected with a neomycin marker gene (Chen and Okayama, 1987), and rat hepatocytes were transfected with a variety of marker genes (Rippe *et al.,* 1990).

### 5. DEAE-Dextran

As disclosed herein, a nucleic acid is delivered into a cell using DEAE-dextran followed by polyethylene glycol. In this manner, reporter plasmids were introduced into mouse myeloma and erythroleukemia cells (Gopal, 1985).

### VI. Cell culturing

Generally, cells of the present invention are cultured in a culture medium, which is a nutrient-rich buffered solution capable of sustaining cell growth.

Culture media suitable for isolating, expanding and differentiating stem cells according to the method described herein include but not limited to high glucose Dulbecco's Modified Eagle's Medium (DMEM), DMEM/F-15, Liebovitz L-15, RPMI 1640, Iscove's modified Dubelcco's media (IMDM), and Opti-MEM SFM (Invitrogen Inc.). Chemically Defined Medium comprises a minimum essential medium such as Iscove's Modified Dulbecco's Medium (IMDM) (Gibco), supplemented with human serum albumin, human Ex Cyte lipoprotein, transfemin, insulin, vitamins, essential and non essential amino acids, sodium pyruvate, glutamine and a mitogen is also suitable. As used herein, a mitogen refers to an agent that stimulates cell division of a cell. An agent can be a chemical, usually some form of a protein that encourages a cell to commence cell division, triggering mitosis. As disclosed herein, serum free media such as those described in U.S. Ser. No. 08/464,599 and WO96/39487, and the "complete media" as described in U.S. Pat. No. 5,486,359 are contemplated for use with the method described herein. It is also disclosed that the culture medium may be supplemented with 10% Fetal Bovine Serum (FBS), human autologous serum, human AB serum or platelet rich plasma supplemented with heparin (2U/ml). Cell cultures maybe maintained in a CO₂ atmosphere, *e.g*., 5% to 12%, to maintain pH of the culture fluid, incubated at 37°C in a humid atmosphere and passaged to maintain a confluence below 85%.

Pluripotent stem cells to be differentiated may be cultured in a medium sufficient to maintain the pluripotency. Culturing of induced pluripotent stem (iPS) cells generated can use various medium and techniques developed to culture primate pluripotent stem cells, more specially, embryonic stem cells, as described in U.S. Pat. App. 20070238170 and U.S. Pat. App. 20030211603. For example, like human embryonic stem (hES) cells, iPS cells can be maintained in 80% DMEM (Gibco #10829-018 or #11965-092), 20% defined fetal bovine serum (FBS) not heat inactivated, 1% non-essential amino acids, 1 mM L-glutamine, and 0.1 mM .beta.-mercaptoethanol. Alternatively, ES cells can be maintained in serum-free medium, made with 80% Knock-Out DMEM (Gibco #10829-018), 20% serum replacement (Gibco #10828-028), 1% non-essential amino acids, 1 mM L-glutamine, and 0.1 mM .beta.-mercaptoethanol. Just before use, human bFGF maybe added to a final concentration of .about 4 ng/mL (WO 99/20741).

### VII. Differentiating Cells to a Desired Tissue Type

Once the pluripotent stem cells have been introduced with the exeogenous expression cassettes designed for condition-responsive expression in the test cell population, the population can be bulked up to any extent required, and then differentiated at will into the desired tissue type.

### B. Liver Cells

Hepatocytes can be differentiated from pluripotent stem cells such as hES cells using an inhibitor of histone deacetylase, as described in U.S. Pat. No. 6,458,589 and PCT publication WO 01/81549 (Geron Corporation). Undifferentiated pluripotent stem cells cells may be cultured in the presence of an inhibitor of histone deacetylase. In an exemplary method, differentiation is initiated with 1% DMSO, then with 2.5 mM of the histone deacetylase inhibitor n-butyrate. The cells obtained can be matured by culturing 4 days in a hepatocyte culture medium containing n-butyrate, DMSO, plus growth factors such as.EGF, hepatocyte growth factor, and TGF-α.

Staged protocols for differentiating pluripotent stem cells such as hES cells into hepatocytes are described in US 2005/0037493 A1 (Geron Corp.). Cells are cultured with several combinations of differentiation and maturation agents in sequence, causing the pluripotent stem cells such as hES cells to differentiate first into early endoderm or hepatocyte precursors, and then to mature hepatocyte-like cells.

Differentiation into endoderm-like cells can be initiated using either butyrate, DMSO or fetal bovine serum, optionally in combination with fibroblast growth factors. Differentiation can then continue using a commercially available hepatocyte culture medium, including factors such as hepatocyte growth factor (HGF), epidermal growth factor (EGF), and/or bone morphogenic protein (*e.g*., BMP-2, 4, or 7) in various combinations. Final maturation may be enhanced by the presence of agents such as dexamethazone or Oncostatin M. An illustration of the "DMSO Protocol" from US 2005/0037493 A1, as applied to the reporter hepatocytes of this invention, is provided below in Example 3. In a refined hepatocyte differentiation protocol, differentiation is initiated using a protein with Activin activity, typically in the presence of or sequentially with other factors like butyrate and/or DMSO (Example 6). The cells can then be matured in stages, using HGF, EGF, and/or BMP, enhanced by the presence of agents such as dexamethazone followed by Oncostatin M.

Hepatocytes can be made by culturing pluripotent stem cells or other non-hepatocytes in a medium under conditions that increase the intracellular level of hepatocyte programming factors to be sufficient to promote programming of the cells into hepatocytes (see U.S. Application No. 61/323,689). The medium may also contain one or more hepatocyte differentiation and maturation agents, like various kinds of growth factors. However, by increasing the intracellular level of hepatocyte programming transcription factors, it is
disclosed to bypass most stages toward mature hepatocytes without the need to change the medium for each of the stages. Therefore, in view of the advantages provided by the present invention, in particular aspects, the medium for culturing cells under hepatocyte programming may be essentially free of one or more of the hepatocyte differentiation and maturation agents, or may not undergo serial change with media containing different combination of such agents.

These agents may either help induce cells to commit to a more mature phenotype-or preferentially promote survival of the mature cells-or have a combination of both these effects. Hepatocyte differentiation and maturation agents illustrated in this disclosure may include soluble growth factors (peptide hormones, cytokines, ligand-receptor complexes, and other compounds) that are capable of promoting the growth of cells of the hepatocyte lineage. Non-limiting examples of such agents include but are not limited to epidermal growth factor (EGF), insulin, TGF-α, TGF-β, fibroblast growth factor (FGF), heparin, hepatocyte growth factor (HGF), Oncostatin M (OSM), IL-1, IL-6, insulin-like growth factors I and II (IGF-I, IGF-2), heparin binding growth factor 1 (HBGF-1), and' glucagon. The skilled reader will already appreciate that Oncostatin M is structurally related to Leukemia inhibitory factor (LIF), Interleukin-6 (IL-6), and ciliary neurotrophic factor (CNTF).

An additional example is n-butyrate, as described in previous patent disclosures (U.S. Pat. No. 6,458,589, U.S. Pat. No. 6,506,574; WO 01/81549). Homologs of n-butyrate can readily be identified that have a similar effect, and can be used as substitutes in the practice of this invention. Some homologs have similar structural and physicochemical properties to those of n-butyrate: acidic hydrocarbons comprising 3-10 carbon atoms, and a conjugate base selected from the group consisting of a carboxylate, a sulfonate, a phosphonate, and other proton donors. Examples include isobutyric acid, butenoic acid, propanoic acid, other short-chain fatty acids, and dimethylbutyrate. Also included are isoteric hydrocarbon sulfonates or phosphonates, such as propanesulfonic acid and propanephosphonic acid, and conjugates such as amides, saccharides, piperazine and cyclic derivatives. A further class of butyrate homologs is inhibitors of histone deacetylase. Non-limiting examples include trichostatin A, 5-azacytidine, trapoxin A, oxamflatin, FR901228, cisplatin, and MS-27-275. Another class of agents is organic solvents like DMSO. Alternatives with similar properties include but are not limited to dimethylacetamide (DMA), hexmethylene bisacetamide, and other polymethylene bisacetamides. Solvents in this class are related, in part, by the property of increasing membrane permeability of cells. Also of interest are solutes such as nicotinamide.

The term "hepatocyte" or "hepatocyte lineage cell" as used in this disclosure means a cell that has one or more, preferably at least three, and more preferably five or seven of the following characteristics: α₁ -antitrypsin; asialoglycoprotein, glycogen storage, cytochrome P450 enzyme expression; glucose-6-phosphatase activity, low to negligible α-fetoprotein, and morphological features of hepatocytes (cuboidal cells, possibly with canalicular spaces between them). Other features of mature hepatocytes isolated from human liver may be present, but are not required to qualify cells as hepatocytes within this definition. Assay methods for identifying cell markers are detailed in U.S. Pat. No. 6,458,589. A "hepatocyte" as described hereinmay be but is not necessarily obtained by differentiating human embryonic stem cells, unless this is explicitly required.

In the context of drug screening, the user may also wish to test the activity of particular drug metabolizing enzymes, such as cytochrome P450 enzymes. A convenient way of surveying the activity of cytochrome P450 is to combine the cells with a "cassette" of substrates: such as midazolam (metabolized by CYP3A4), tolbutamide (metabolized by CYP2C9), phenacetin (CYP1A2), and bufuralol (CYP2D6). Activity can be quantitated as being about 0.1, 1, or 10 times that of a reference cell line, such as HepG2 cells. A convenient way of monitoring metabolites of all the drugs in the cassette simultaneously is by GCMS. If desirable, the cells can be treated with compounds such as dexamethazone or Rifampicin before or during use in drug screening, so as to increase cytochrome P450 expression or activity in the cells.

### C. Nerve Cells

Neural cells can be generated from pluripotent stem cells such as hES cells according to the method described in U.S. Pat. No. 6,833,269; Carpenter *et al.,* 2001; and WO 03/000868 (Geron Corporation). Undifferentiated hES cells or embryoid body cells are cultured in a medium containing one or more neurotrophins and one or more mitogens, generating a cell population in which at least ∼60% of the cells express A2B5, polysialylated NCAM, or Nestin and which is capable of at least 20 doublings in culture. Exemplary mitogens are EGF, basic FGF, PDGF, and IGF-1. Exemplary neurotrophins are NT-3 and BDNF. The use of TGF-β Superfamily Antagonists, or a combination of cAMP and ascorbic acid, can be used to increase the proportion of neuronal cells that are positive for tyrosine hydroxylase, a characteristic of dopaminergic neurons. The proliferating cells can then be caused to undergo terminal differentiation by culturing with neurotrophins in the absence of mitogen.

Oligodendrocytes can be generated from pluripotent stem cells such as hES cells by culturing them as cell aggregates, suspended in a medium containing a mitogen such as FGF, and oligodendrocyte differentiation factors such as triiodothyronine, selenium, and retinoic acid. The cells are then plated onto a solid surface, the retinoic acid is withdrawn, and the population is expanded. Terminal differentiation can be effected by plating on poly-L-lysine, and removing all growth factors. Populations can be obtained in which over 80% of the cells are positive for oligodendrocyte markers NG2 proteoglycan, A2B5, and PDGFRα, and negative for the neuronal marker NeuN. See PCT publication WO 04/007696 and Keirstead *et al.,* 2005. Derivation of retinal pigment epithelial cells has also been reported (Klimanskaya *et al.,* 2004).

### D. Heart Cells

Cardiomyocytes or cardiomyocyte precursors can be generated from pluripotent stem cells such as hES cells according to the method provided in WO 03/006950. The cells are cultured in suspension with fetal calf serum or serum replacement, and optionally a cardiotrophic factor that affects DNA-methylation, such as 5-azacytidine. Alternatively, cardiomyocyte clusters can be generated by culturing on a solid substrate with Activin A, followed by culturing with a bone morphogenic protein like BMP4, and optionally by further culturing with an insulin-like growth factor like IGF-1. If desired, spontaneously contracting cells can then be separated from other cells in the population, by density centrifugation.

Further process steps can include culturing the cells so as to form clusters known as Cardiac Bodies™, removing single cells, and then dispersing and reforming the Cardiac Bodies™ in successive iterations. Populations are obtained with a high proportion of cells staining positive for cTnI, cTnT, cardiac-specific myosin heavy chain (MHC), and the transcription factor Nk×2.5. See WO 03/006950, Xu *et al.,* 2002; and US 2005/0214939 A1 (Geron Corporation).

### E. Other Cell Types

Islet cells can be differentiated from pluripotent stem cells such as hES cells (WO 03/050249, Geron Corp.) by initiating differentiation by culturing in a medium containing a combination of several factors selected from Activin A, a histone deacetylase inhibitor (such as butyrate), a mitogen (such as bFGF); and a TGF-β Superfamily antagonist (such as noggin). The cells can then be matured by culturing with nicotinamide, yielding a cell population in which at least 5% of the cells express Pdx1, insulin, glucagon, somatostatin, and pancreatic polypeptide. Cell clusters may form buds enriched for insulin producing cells, which can be recovered by filtering. See WO 03/050249 (Geron Corp.).

Hematopoietic cells can be made by coculturing pluripotent stem cells such as hES cells with murine bone marrow cells or yolk sac endothelial cells was used to generate cells with hematopoietic markers (U.S. Pat. No. 6,280,718). Hematopoietic cells can also be made by culturing stem cells with hematogenic cytokines and a bone morphogenic protein, as described in US 2003/0153082 A1 and WO 03/050251 (Robarts Institute).

Mesenchymal progenitors and fibroblasts can be generated from pluripotent stem cells such as hES cells according to the method described in WO 03/004605. hES-derived mesenchymal cells can then be further differentiated into osteoblast lineage cells in a medium containing an osteogenic factor, such as bone morphogenic protein (particularly BMP4), a ligand for a human TGF-β receptor, or a ligand for a human vitamin D receptor (WO 03/004605; Sotile *et al.,* 2003). US 2004/0009589 A1 (Iskovitz-Elder *et al.*) and US 2003/0166273 A1 (Kaufman *et al.,* Wisconsin) report endothelial cells derived from human embryonic stem cells. Chondrocytes or their progenitors can be generated by culturing stem cells in microaggregates with effective combinations of differentiation factors listed in WO 03/050250 (Geron Corp.).

Other differentiation methods known in the art or subsequently developed can be used in conjunction with this disclosure to create engineered cells representative of other tissues.

### VIII. Screening platform and methods

The engineered cell population or cells derived therefrom in certain aspects of the invention can be used in a variety of applications. These include but not limited to study biological response or drug response; screening cytotoxic compounds, carcinogens, mutagens growth/regulatory factors, pharmaceutical compounds, *etc., in vitro;* elucidating the mechanism or conditions of cell programming or development pathways; studying the mechanism by which drugs and/or growth factors operate; and the production of biologically active products, to name but a few.

### B. Test compound screening

Engineered cells or cells derived therefrom of certain aspects of this invention can be used to screen for factors (such as solvents, small molecule drugs, peptides, and polynucleotides) or environmental conditions (such as culture conditions or manipulation) that affect the expression characteristics of exogenous expression caseettes comprising condition-responsive regulatory elements provided herein.

In some applications, stem cells (differentiated or undifferentiated) are used to screen factors that promote maturation of cells along a selected cell lineage such as the hepatocyte lineage, or promote proliferation and maintenance of such cells in long-term culture. For example, candidate hepatocyte maturation factors or growth factors are tested by adding them to stem cells in different wells, and then determining any phenotypic change that results, according to desirable criteria for further culture and use of the cells.

Particular screening applications of this invention relate to the testing of pharmaceutical compounds in drug research. The reader is referred generally to the standard textbook *In vitro* Methods in Pharmaceutical Research, Academic Press, 1997, and U.S. Pat. No. 5,030,015). As disclosed herein, cells programmed to the hepatocyte lineage play the role of test cells for standard drug screening and toxicity assays, as have been previously performed on hepatocyte cell lines or primary hepatocytes in short-term culture. Assessment of the activity of candidate pharmaceutical compounds generally involves combining the hepatocytes provided in certain aspects of this disclosure with the candidate compound, determining any change in the morphology, marker phenotype, or metabolic activity of the cells that is attributable to the compound (compared with untreated cells or cells treated with an inert compound), and then correlating the effect of the compound with the observed change. The screening may be done either because the compound is designed to have a pharmacological effect on liver cells, or because a compound designed to have effects elsewhere may have unintended hepatic side effects. Two or more drugs can be tested in combination (by combining with the cells either simultaneously or sequentially), to detect possible drug-drug interaction effects.

### 2. Toxicity Testing

Use of the cells of this invention containing condition-responsive expression cassettes in toxicity testing involves combining the cell population with the agent to be screened (typically by adding it to the medium). Examples of such a agent include, but need not be limited to, pharmaceutical compounds, agricultural chemicals, specialty chemicals, cosmetics and food additives. The effect of the agent on the exogenous expression cassette is followed typically by comparing the signal from the marker gene in the presence and absence of the agent, using a detection system appropriate for the selectable or screenable marker chosen.

By way of illustration, iPS cells are genetically modified and differentiated to create a population of hepatocytes containing a promoter for heme oxygenase 1, linked to a green fluorescent protein reporter gene. The cells are combined with the test agent in the same medium, and fluorescence is measured in comparison with fluorescence in the absence of the test agent. Increase in fluorescence level indicates that the heme oxygenase 1 gene is up-regulated, apparently in response to oxidative stress induced by the test agent. Different agents and agent combinations can be screened in a rapid throughput process, for example, by establishing the cells in the wells of a microtiter plate. Agents tested according to this system can be identified and selected for further development, testing, or use because they do not cause substantial increase or alteration in the level of reporter expression (which means that if there is any effect attributable to the presence of the test agent, it is below a threshold that the user considers acceptable).

Depending on the differentiation protocol, cell populations can be used that are at least 50%, 80%, or 90% homogeneous for the cell type of interest. Where the cell populations are relatively pure, or when the selected promoter is only active in the cell type of interest (e.g., the CYP3A4 promoter in hepatocytes), then effects of the test agent on the target cell can be measured simply by following signal from the reporter gene in the cell population as a whole.

However, when the cell populations are more heterogeneous, and the promoter can be induced in more than one of the cell types present, then it may be preferable to follow the effect on a cell-by-cell basis. A cell that contains both a metabolic-responsive expression cassette and a tissue-specific expression cassette is equipped to do this particularly well. The test agent is combined with the cell population as a whole, but the output of the assay is measured as a change in the metabolic-responsive marker when present in a cell labeled with the tissue specific expression cassette. A benefit of this approach is that there is no need for the target cell type to predominate the reagent cell population. Populations comprising less than 20%, 10%, or 5% of the target cells can be used, since a drug-induced effect will be demonstrated if there are detectable cells in which both markers are expressed. This enables the drug screening techniques of this invention to be used with relatively rare cell types or subtypes-*e.g*., insulin-producing pancreatic islet cells, or neural cells that utilize a particular neurotransmitter.

Cell populations equipped with a plurality of metabolic or toxicologically responsive expression cassette (either as different exogenous expression cassettes in a single cell line, or in a population of mixed cells containing different exogenous expression cassettes) can be used to monitor multiple assault pathways simultaneously, as long as the products of the selectable or screenable marker genes are distinguishable.

In some particular applications, compounds are screened initially for potential hepatotoxicity (Castell *et al.,* 1997). Cytotoxicity can be determined in the first instance by the effect on cell viability, survival, morphology, and leakage of enzymes into the culture medium. More detailed analysis is conducted to determine whether compounds affect cell function (such as gluconeogenesis, ureogenesis, and plasma protein synthesis) without causing toxicity. Lactate dehydrogenase (LDH) is a good marker because the hepatic isoenzyme (type V) is stable in culture conditions, allowing reproducible measurements in culture supernatants after 12-24 h incubation. Leakage of enzymes such as mitochondrial glutamate oxaloacetate transaminase and glutamate pyruvate transaminase can also be used. Gomez-Lechon *et al.* (1996) describes a microassay for measuring glycogen, which can be used to measure the effect of pharmaceutical compounds on hepatocyte gluconeogenesis.

Other current methods to evaluate hepatotoxicity include determination of the synthesis and secretion of albumin, cholesterol, and lipoproteins; transport of conjugated bile acids and bilirubin; ureagenesis; cytochrome p450 levels and activities; glutathione levels; release of α-glutathione s-transferase; ATP, ADP, and AMP metabolism; intracellular K⁺ and Ca²⁺ concentrations; the release of nuclear matrix proteins or oligonucleosomes; and induction of apoptosis (indicated by cell rounding, condensation of chromatin, and nuclear fragmentation). DNA synthesis can be measured as [³H]-thymidine or BrdU incorporation. Effects of a drug on DNA synthesis or structure can be determined by measuring DNA synthesis or repair. [³H]-thymidine or BrdU incorporation, especially at unscheduled times in the cell cycle, or above the level required for cell replication, is consistent with a drug effect. Unwanted effects can also include unusual rates of sister chromatid exchange, determined by metaphase spread. The reader is referred to Vickers (1997) for further elaboration.

### 3. Screening for Positive Pharmacological Effect

Besides screening test compounds for toxicology, drug metabolism, and disposition, the cells of this invention can also be used to screen for positive pharmacological effect. For example, pancreatic cells containing a selectable or screenable marker system driven by an insulin promoter can be used to screen drugs capable of inducing insulin secretion. Neuronal cells containing a selectable or screenable marker system driven by promoters for genes in neurotransmitter synthesis, release, or uptake can be used to screen drugs with a potentially beneficial neurological effect. The use of cells, kits, and methodology of this invention for positive screening parallels that of toxicity testing, selecting appropriate promoter constructs and adapting the assays as appropriate.

In another example, compounds can be tested for cytoprotection against another drug or culture condition. For example, cells containing a exogenous expression cassette for a gene upregulated in apoptosis or stress (like PUMA or heme oxygenase 1) are cultured in the presence of stressors such as menadione, tertiary butylhydroquinone (TBHQ), hydroperoxidase, quinone, or abnormal oxygen levels to turn on the selectable or screenable marker signal. Once established, cells cultured with such stressors can be used to screen drugs that will prevent, lower, or reverse selectable or screenable marker signaling, thereby denoting a lower level of gene expression, and hence a protective effect. This can be used with pluripotent stem cell-derived cardiomyocytes, for example, to test drugs for suitability in treating cardiac ischemia. In tandem with screening of drugs for positive effects, matched populations of hepatocyte reporter cells can be used to screen for toxicological effects of the same compounds.

### 4. Validation of Drug Targets and Drug Metabolizing Enzymes

During the course of screening for a toxicological or pharmaceutical effect, the user may wish to validate the presumed target of a particular drug, or an enzyme believed to be involved in its metabolism. This can be done by combining the drug with exogenous expression cassette-containing cells in the presence or absence of a substance that either activates or inhibits transcription or translation of the drug target or metabolizing enzyme. The exogenous expression cassette is chosen to reflect gene activity downstream from the activity being tested. The user then determines whether there is a difference in expression of the selectable or screenable marker gene in the presence of the drug with or without the RNAi, as an indication of whether the drug does influence the drug target or enzyme in question.

Suitable inhibitors for use in this context are RNA molecules (RNAi) of the single or double stranded variety, having a sequence that enables it to inactivate translation in a gene specific manner. The synthesis and use of RNAi molecules and other inhibitors suitable for use in this context are well described in the art. See, for example, Huan et al., Cancer Res. 64:4294, 2004; Chan et al., 2005; Manoharan, 2004; WO 04/094595; WO 05/014782). Other suitable activators and inhibitors include small molecule drugs known to upregulate or downregulate the gene at the transcription level (Campbell *et al.,* 1996).

Known drug targets include G protein-coupled receptors (GPCRs), activated by ligands like TNF; peroxisome proliferation-activated receptors (PPARs), which binds muraglitazar and other compounds; cytochrome P450 regulators like PXR, which are activated by dexamethazone, Rifampicin, or pregnenalone 16α-carbonitrile; the nuclear receptor CAR, which are activated by phenobarbital and other barbiturates; Phase II enzymes like glycosyl transferase, which process polychlorinated biphenyl compounds; aryl hydrocarbon (Ah) receptors, which bind benzo[a]pyrene and β-naphthoflavone; and estrogen receptors, which bind estrogen analogs like tamoxifen.

Known drug metabolizing enzymes include the cytochrome P450 system (Ortiz de Montellano *et al.,* supra), N-acetyl transferase, and enzymes involved in conjugation of bile acids and other compounds.

To illustrate this aspect of the invention, drug metabolism in the liver can be studied using hepatocytes having an exogenous expression cassette that responds to oxidative stress. A drug that is metabolized through the cytochrome P450 system (e.g., phenobarbital) can be combined with the cells in the presence and absence of RNAi specific for particular P450 enzymes like CYP3A4. If there is higher selectable or screenable marker activity induced by the drug in the presence of the RNAi, then the reduction in CYP3A4 activity caused by the RNAi is resulting in increased stress-implicating CYP3A4 in the metabolic pathway of the drug.

- In a similar fashion, role of the estrogen receptor in the pharmaceutical activity of a drug can be evaluated using cells having an exogenous expression cassette that reflects transcription of a gene up-regulated by estrogen. If there is lower selectable or screenable marker activity induced by the drug in the presence of RNAi specific for the estrogen receptor, then the estrogen receptor is validated as a target for the drug being tested.

### 5. Effect on Allelic Variants

Exogenous expression cassette-containing cells made from the same pluripotent stem cell line but engineered to contain different variants of a drug metabolizing enzyme can be used to compare the processing or effect of a drug thought to be metabolized by the enzyme. For example, hepatocytes derived from the same iPS cell having the usual form of the CYP2D6 gene, can be compared with hepatocytes having the variant present in 6% of the population for the effect of a drug like dextromethorphan. Differences in drug metabolism attributable to the variation will affect the signal generated through an exogenous expression cassette that responds to metabolic or toxicologic changes in the cell, or reflects expression of a gene product implicated in metabolism of the drug.

In a similar fashion, cells engineered to contain different variants of a drug target can be used to compare the effect of a drug on the target variants. For example, neuronal cells having variations in an enzyme involved in uptake of a neurotransmitter can be compared for the effect of a drug known to affect uptake (e.g., bupropion). Differences in the pharmacological effect of the drug attributable to the variation will affect the signal generated through an exogenous expression cassette that responds to presence of the neurotransmitter.

Separate cell populations having different variants of the drug target or drug metabolizing enzyme can be tested with the drug in parallel. Optionally, each variant can be placed in a cell population having different selectable or screenable marker genes. This enables the user to combine the two cell populations, and measure the effect of the drug on both variants together.

### C. Cells and methods for testing programming

To aid identification of desired cell types, the cells that comprise a cell-specific or tissue-specific marker expression cassette may be used to test programming conditions, more particularly, differentiation conditions. The expression cassette may comprise a selectable or screenable marker operably linked to a transcriptional regulatory element specific for the desired cell types. For example, the expression cassette may comprise a hepatocyte-specific promoter for hepatocyte production, isolation, selection, or enrichment.

Therefore, the ability of a particular candidate gene or a combination of candidate genes to act as programming factors for a specific cell type, such as hepatocytes or novel cell types that have never been made from programming such as differentiation of pluripotent stem cells, can be tested using the methods and cells provided in this disclosure. Efficacy of particular candidate genes or combinations of candidate genes in programming can be assessed by their effect on cell morphology, marker expression, enzymatic activity, proliferative capacity, or other features of interest, which is then determined in comparison with parallel cultures that did not include the candidate genes or combinations. Candidate genes may be transcription factors important for differentiation into desired cell types or for function of the desired cell types.

As disclosed herein, starting cells, such as pluripotent stem cells comprising condition-responsive expression cassettes, may further comprise at least one expression cassette for expression of a candidate gene or a combination of candidate genes. The candidate expression cassette may comprise an externally controllable transcriptional regulatory element, such as an inducible promoter. The activity of these promoters may be induced by the presence or absence of biotic or abiotic factors. Inducible promoters are a very powerful tool in genetic engineering because the expression of genes operably linked to them can be turned on or off at certain stages of development of an organism or in a particular tissue. Tet-On and Tet-Off inducible gene expression systems based on the essential regulatory components of the *E. coli* tetracycline-resistance operon may be used. Once established in the starting cells, the inducer doxycycline (Dox, a tetracycline derivative) could controls the expression system in a dose-dependent manner, allowing to precisely modulate the expression levels of candidate genes.

### VIII. Examples

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice.

### Example 1 - Production of engineered stem cell lines

The inventors contemplated a collection of engineered stem cell lines that can be used as a set to study any biological response in the human body. The base cell line for the construction of the set is an episomally derived iPS cell line made from a human tissue sample. Into this episomal line, a homologous recombination strategy has been used to introduce recombinase recognition sites into the Rosa 26 locus of the parental iPS line. This strategy, disclosed in published US Patent Application No. 20100011455, is illustrated in **FIG. 1****.** The Rosa 26 targeting cassette was made so as to insert in between intron I and II in the native Rosa 26 locus on human chromosome 3. The cassette included, in 5' to 3' sequence, a 5' homologous arm for targeting, a spacer, a recombinase recognition site (white triangle), a protein coding sequence from the thymidine kinase gene beginning with an ATG to start transcription, a 2A sequence, a second protein coding sequence for an antibiotic resistance gene for resistance to neomycin, a second recombinase recognition site (black triangle) and a 3' homologous arm. The cassette is designed to be introduced into cells of the iPS line, with successful desired recombinant events being identified by resistance to neomycin. This cassette has been successfully transferred into the Rosa 26 locus of an episomally reprogrammed iPS line. This basal Rosa 26 knock-in line has been verified by PCR, expanded and banked in aliquots.

With the basal Rosa 26 knock-in iPS line in hand, it then becomes convenient to introduce any desired genetic construct into this site. Since the Rosa 26 locus is expressed in essentially all tissues, expression at the locus is not repressed regardless of the cell lineage into which the iPS line is differentiated.

Also shown in **FIG. 1** are the elements of a secondary engineered iPS line, made from this basal Rosa 26 iPS line. This particular line is constructed for selection of hepatocytes. First, a genetic construct was assembled which contained two expression cassettes, one cassette to permit selection of the desired recombinant event, and one cassette to permit tissue specific selection of the desired tissue type, *i.e.,* hepatocytes. At the 5' end of the construct, there was a left recombination recognition site, followed by a protein coding sequence for another antibiotic resistance gene, designated herein as the iPS selector. This coding sequence is driven by the native Rosa 26 promoter to permit successful desired recombinant cells to be identified by resistance to the antibiotic for which the iPS selector confers resistance. Also in the construct, oriented in the opposite direction, is a construct including the promoter of alpha-1 antitrypsin (pAAT), which drives the expression of a second antibiotic selection gene, this one to be used to select cells when the cells have differentiated into hepatocytes. In this particular construct, there are also several enhancer elements (designated as ApoEl-4) which have been found to enhance the expression level of this particular promoter in hepatocytes. This construct has been built, transfected into the basal Rosa 26 iPS line, and antibiotic resistant colonies have been recovered. Subsequent characterization will identify the proper insertion events and those clones will be expanded and banked.

This same strategy can then be used to make each of the lines in the collection of lines envisioned herein. Shown in **FIG. 2** is an example of the common format of design of the genetic constructions to go into the iPS lines of the collection. For each insertion, there is an iPS selector which permits selection of the desired recombinant insertion. For each insertion, there is a tissue specific promoter, the promoters being different in different elements of the set, but each of the promoters selected for tissue specific expression. The tissue specific expression will be in some instances an organ, *e.g*., pan cardiac, in some instances an organ subtype, *e.g*., atrial cell, in some cases a body wide cell type, *e.g.,* endothelial cell, or in some instances a level of differentiation, *e.g.,* a cardiac progenitor. The tissue specific promoter actuates expression of a second gene for resistance to a second antibiotic resistance gene, labeled a cell type selector in **FIG. 2****.** The cell type selector is used to purify the cells of interest by enabling the survival of the cells which express the tissue specific promoter. A marker gene, such as a fluorescent protein, luciferase, a proprietary marker system, such as HaloTag or SNAP, is linked in expression to the cell type selector by a 2A linker, which works to co-express two distinct proteins driven by a common promoter. The collection will have a large number of different iPS cell lines, each engineered with a different tissue specific promoter element so that each line either reports (fluorescence) or is selectable (antibiotic resistance), or both, when the conditional responsive promoter element in its construct is active.

With this set of lines each of which is pre-engineered to become purifiable differentiated cells of a selected lineage, it then becomes possible to tag or mark any desired drug target, cell receptor or pathway in the cells. The number of known druggable target and pathways of interest to the pharmaceutical industry is reasonably small, less than 100 pathways and targets. Vectors for each of those targets and pathways will be assembled into piggyBac vectors which contain genetic constructs that will exhibit a marker gene, such as a second distinct fluorescent marker, when the target or pathway is active in the cell. piggyBac vectors can readily be transformed efficiently into iPS cells without silencing and clones containing the piggyBac vectors can readily be identified which have appropriate expression of conditionally responsive promoters in the piggyBac vectors. The set of piggyBac vectors can then be mixed and matched as needed with the set of iPS lines. The result is that the set of iPS lines permits differentiation and purification of any cell type in the human body for which a tissue specific promoter can be identified and the use of the piggyBac vector permits screening for any druggable target or pathway in those cells. This system thus enables drug screening to be done on any cell type in the human body on any target that a pharmaceutical discovery effort might desire. All of the cells of the body and all of the pathways in those cells are now available for drug discovery in the most appropriate biological context possible outside of the human body itself.

Another use for this set of iPS lines is for the discovery of differentiation processes. As the science of stem cells advances, slowly methods are being found to differentiate stem cells into many differentiated cell types. The tool involving a set of iPS lines as described above enables that process to be dramatically accelerated. By using an iPS line which will express its inserted marker gene when the cell differentiates into a given progeny cell, it now becomes possible to perform random or semi random screens on differentiation conditions, since any condition which causes the undifferentiated stem cells to differentiate into the target cells of interest can be detected by the activation of the marker gene of **FIG. 2****.** Once a single differentiation method is identified, even if it works at low efficiency, the same tool permits recursive experimentation on the initial method to be performed to increase efficiency and yield, while at all levels of efficiency of the process, purified cultures of the cells of interest can be simply obtained by antibiotic purification. So developing processes to produce purified cultures of any cell type in the body is now possible.

Another use for the set of tool lines is for use as an assay of developmental toxicology. Since the iPS cells will exhibit the marker gene only when they differentiate into the target cell type, once a differentiation process is working at some level of efficiency, it is then possible to pertubate that process, by adding molecules which are potential developmentally toxic, to see if the molecules influence the yield of the target cells. For example, using the hepatocyte (liver) example of **FIG. 1****,** under processes favoring the differentiation of hepatocytes, the cell line of **FIG. 1** will yield anywhere from 20 to 70% hepatocytes, the efficiency level of which can be measured by the observed fluorescence from the cells as they become hepatocytes. For any process, the level will vary somewhat, but vary within limits about a statistical norm. It then becomes possible to set up that process in multiwell culture plates and to add a potential teratogen or other potential developmentally toxic agent to each well. The wells that fail to produce the normal yield of hepatocytes would indicate that the agent used in those particular wells is potentially harmful to the development of that cell type. This system can be replicated for many different cell types to identify those known or new agents which interfere with any form of developmental biology.

The most exhaustive variant would be a set of perhaps 10,000 lines where every promoter in the body which is differentially expressed in any cell type is included (*i.e.,* excluding all promoters express similarly in all or most cells). This set could then be used to follow the expression characteristics of any promoter in any developmental pathway.

This design would enable high-throughput screening using the luciferase reporter, high-content imaging or high-throughput FACS using the GFP reporter, or purification using the antibiotic resistance gene, the expression of all of which is controlled by the genetic regulatory element in the genetic construct.

In addition, examination of the variation in gene expression patterns of particular cell types can be examined with this tool. The inventors will construct a set of iPS cell lines with all the promoters from all the liver P450 genes. Then the iPS cells will be differentiated to hepatocytes and be used to track the spectrum of P450 responses to an applied drug.

### REFERENCES

The following references provide exemplary procedural or other details supplementary to those set forth herein.
U.S. Patent 5,030,015
U.S. Patent 5,302,523
U.S. Patent 5,322,783
U.S. Patent 5,384,253
U.S. Patent 5,460,964
U.S. Patent 5,464,765
U.S. Patent 5,486,359
U.S. Patent 5,486,359
U.S. Patent 5,486,359
U.S. Patent 5,538,877
U.S. Patent 5,538,880
U.S. Patent 5,538,880
U.S. Patent 5,550,318
U.S. Patent 5,550,318
U.S. Patent 5,563,055
U.S. Patent 5,563,055
U.S. Patent 5,580,859
U.S. Patent 5,589,466
U.S. Patent 5,591,616
U.S. Patent 5,610,042
U.S. Patent 5,610,042
U.S. Patent 5,635,387
U.S. Patent 5,656,610
U.S. Patent 5,677,136
U.S. Patent 5,681,599
U.S. Patent 5,702,932
U.S. Patent 5,716,827
U.S. Patent 5,736,396
U.S. Patent 5,736,524
U.S. Patent 5,750,397
U.S. Patent 5,759,793
U.S. Patent 5,780,448
U.S. Patent 5,789,215
U.S. Patent 5,811,094
U.S. Patent 5,827,735
U.S. Patent 5,827,740
U.S. Patent 5,837,539
U.S. Patent 5,837,670
U.S. Patent 5,925,565
U.S. Patent 5,935,819
U.S. Patent 5,945,100
U.S. Patent 5,981,274
U.S. Patent 5,994,136
U.S. Patent 5,994,624
U.S. Patent 6,013,516
U.S. Patent 6,280,718
U.S. Patent 6,458,589
U.S. Patent 6,458,589
U.S. Patent 6,458,589
U.S. Patent 6,506,574
U.S. Patent 6,833,269
U.S. Patent 6,833,269
U.S. Patent 6,991,897
U.S. Patent 7,015,037
U.S. Patent 7,399,632
U.S. Patent 7,410,773
U.S. Patent 7,410,798
U.S. Patent 7,422,736
U.S. Appln. 61/323,689
U.S. Appln. 08/464,599
U.S. Appln. 61/172,079
U.S. Appln. 61/184,546
U.S. Publn. 2003/0003573
U.S. Publn. 2003/0153082 A1
U.S. Publn. 2003/0166273 A1
U.S. Publn. 20030211603
U.S. Publn. 2004/0009589 A1
U.S. Publn. 2005/0037493 A1
U.S. Publn. 2005/0214939 A1
U.S. Publn. 20070238170
U.S. Publn. 2010/0003757
U.S. Publn. 2010/0011455
Alison et al, Hepatol., 29:678-83, 1998.
Amit et al., Dev. Bio., 227:271-278, 2000.
Andrews et al., In: Teratocarcinomas and Embryonic Stem Cells, Robertson (Ed.), IRL Press, 207-246,1987.
Ausubel et al., Current Protocols in Molecular Biology, Greene Publ. Assoc. Inc. & John Wiley & Sons, Inc., MA, 1996.
Ausubel et al., In: Current Protocols in Molecular Biology, John, Wiley & Sons, Inc, New York, 1994.
Bajic et al., Bioinformatics, 18:198-199, 2002.
Blomer et al., J. Virol., 71(9):6641-6649, 1997.
Boyer et al., Cell, 122(6):947-56, 2005.
Byrne et al., Nature, 450(7169):497-502, 2007.
Campbell et al., J. Cell Sci., 109:2619, 1996
Carpenter et al., Exp. Neurol., 172(2):383-97, 2001.
Cassiede et al., J. Bone Miner. Res., 11(9):1264-1273, 1996.
Castell et al., In: In vitro Methods in Pharmaceutical Research, Academic Press, 375-410, 1997.
Chambers et al., Cell, 113(5):643-55, 2003.
Chan et al., Drug Discov. Today, 10:587, 2005.
Chen and Okayama, Mol. Cell Biol., 7(8):2745-2752, 1987.
Current Protocols in Stem Cell Biology, Bhatia et. al. (Ed.), John Wiley and Sons, Inc., 2007. deFelipe, Curr. Gene Ther., 2:355-378, 2002.
European Patent EP0412700
European Patent EP1507865
Evans, et al., In: Cancer Principles and Practice of Oncology, Devita et al. (Eds.), Lippincot-Raven, NY, 1054-1087, 1997.
Fechheimer et al., Proc Natl. Acad. Sci. USA, 84:8463-8467, 1987.
Ferry et al., Hum. Gene Ther., 9(14):1975-81, 1998.
Follenzi et al., Hum. Gene Ther., 13(2):243-60, 2002.
Fraley et al., Proc. Natl. Acad. Sci. USA, 76:3348-3352, 1979*.*
Gehrke et al., Gene, 322:137-43, 2003.
Ghosh and Bachhawat, In: Liver Diseases, Targeted Diagnosis and Therapy Using Specific Receptors and Ligands, Wu et al. (Eds.), Marcel Dekker, NY, 87-104,1991.
Ghosh et al., J. Hepatol., 32(1Suppl):238-52, 2000.
Gomes-Lechon et al., In: In vitro Methods in Pharmaceutical Research, Academic Press, 129-153, 1997.
Gopal, Mol. Cell Biol., 5:1188-1190, 1985.
Graham and Van Der Eb, Virology, 52:456-467, 1973.
Gronthos, Blood, 84(12):41644173, 1994.
Harland and Weintraub, J. Cell Biol, 101(3):1094-1099, 1985.
Hill et al., Exp. Hematol., 24(8):936-943, 1996.
Huan et al., Cancer Res., 64:4294, 2004.
Irion et al., Nat Biotechnol., 25(12):1477-82, 2007
Jaiswal et al., J. Cell Biochem., 64(2):295-312, 1997.
Johnstone *et al.,* 238(1):265-272, 1998.
Kaeppler et al., Plant Cell Rep., 8:415-418, 1990.
Kaneda et al., Science, 243:375-378, 1989.
Kato et al, J. Biol. Chem., 266:3361-3364, 1991.
Keirstead et al., J. Neurosci., 25(19):4694-705, 2005.
Kim et al., Mol. Cell Biol., 12(8):3636-43, 1992.
Klein et al., Nature, 327:70-73, 1987.
Klimanskaya et al., Cloning Stem Cells, 6:217, 2004.
Kramer et al., Mol. Ther., 7(3):375-85, 2003.
Langle-Rouault et al., J. Virol., 72(7):6181-6185, 1998.
Levitskaya et al., Proc. Natl. Acad. Sci. USA, 94(23):12616-12621, 1997.
M. Manoharan, Curr. Opin. Chem. Biol., 8:570, 2004..
Macejak and Sarnow, Nature, 353:90-94,1991.
Makino et al., J. Clin. Invest., 103(5):697-705, 1999.
Maniatis, et al., Molecular Cloning, A Laboratory Manual, Cold Spring Harbor Press, Cold Spring Harbor, N.Y., 1988.
Mann et al., Cell, 33:153-159, 1983.
McBratney et al. Curr. Opin. Cell Biol., 5(6):961-5, 1993.
Miao et al., Trends Biotechnol., 19(9):349-55, 2001.
Miao et al., Mol. Ther., 1(6):522-32,2000.
Miller et al., Am. J. Clin. Oncol., 15(3):216-221, 1992.
Nabel et al., Science, 244(4910):1342-1344, 1989.
Naldini et al., Science, 272(5259):263-267, 1996.
Nicolas and Rubenstein, In: Vectors: A survey of molecular cloning vectors and their uses, Rodriguez and Denhardt, eds., Stoneham: Butterworth, pp. 494-513, 1988.
Nicolau and Sene, Biochim. Biophys. Acta, 721:185-190, 1982.
Nicolau et al., Methods Enzymol., 149:157-176, 1987.
Paris et al., Cancer Epidemiol. Biomarkers Prev., 8:901, 1999.
Paskind et al., Virology, 67:242-248, 1975.
PCT Appln. WO 01/098482
PCT Appln. WO 01/81549
PCT Appln. WO 01/81549
PCT Appln. WO 03/000868
PCT Appln. WO 03/004605
PCT Appln. WO 03/006950
PCT Appln. WO 03/042405
PCT Appln. WO 03/050249
PCT Appln. WO 03/050250
PCT Appln. WO 03/050251
PCT Appln. WO 04/007696
PCT Appln. WO 04/094595
PCT Appln. WO 05/014782
PCT Appln. WO 09/130208
PCT Appln. WO 94/09699
PCT Appln. WO 94/09699
PCT Appln. WO 95/011308
PCT Appln. WO 95/06128
PCT Appln. WO 96/39487
PCT Appln. WO 99/20741
Pelletier and Sonenberg, Nature, 334:320-325, 1988.
Potrykus et al., Mol. Gen. Genet., 199(2):169-177, 1985.
Potten, Philos. Trans. R Soc. Lond. B Biol. Sci., 353:821-30, 1998.
Potter et al., Proc. Natl. Acad. Sci. USA, 81:7161-7165, 1984.
Prestridge, J. Mol. Biol., 249:923-932, 1995
Reubinoff et al., Nat. Biotechnol., 18:399B404, 2000.
Rippe, et al., Mol. Cell Biol., 10:689-695, 1990.
Ryan et al., J. Gener. Virol., 78:699-722, 1997.
Sambrook and Russell, Molecular Cloning: A Laboratory Manual, 3rd Ed. Cold Spring Harbor Lab. Press, 2001.
Scherf et al., J. Mol. Biol., 297:599-606, 2000.
Scymczak et al., Nature Biotech., 5:589-594, 2004.
Simonet et al., J. Biol. Chem., 268(11):8221-9, 1993.
Smith, In: Origins and Properties of Mouse Embryonic Stem Cells, Annu. Rev. Cell. Dev. Biol., 2000.
Sotile et al., Cloning Stem Cells, 5(2):149-55,2003.
Takahashi and Yamanaka, Cell, 126:663-676,2006.
Takahashi et al., Cell, 126(4):663-76, 2007.
Takahashi et al., Cell, 131:861-872, 2007.
Temin, In: Gene Transfer, Kucherlapati (Ed.), NY, Plenum Press, 149-188,1986.
Thomson and Marshall, Curr. Top. Dev. Biol., 38:133-165, 1998.
Thomson and Odorico, J. Trends. Biotechnol., 18:53B57, 2000.
Thomson et al. Proc. Natl. Acad. Scie. USA, 92:7844-7848,1995.
Thomson et al., Science, 282:1145, 1998.
Tur-Kaspa et al., Mol. Cell Biol., 6:716-718, 1986.
Vickers In: In vitro Methods in Pharmaceutical Research, Academic Press, 375-410, 1997.
Watt, Philos. Trans. R. Soc. Lond. B. Biol. Sci., 353:831, 1997.
Webber, In: Pharmacogenetics, Oxford Univ. Press, 1997.
Wilson et al., Science, 244:1344-1346, 1989.
Wolf et al., Br. Med. Bull., 55:366, 1999.
Wolf et al., Br. Med. J., 320:987, 2000.
Wong et al., Gene, 10:87-94, 1980.
Wu and Wu, Biochemistry, 27: 887-892, 1988.
Wu and Wu, J. Biol. Chem., 262:4429-4432, 1987.
Xie et al., Bioinformatics, 22(22):2722-2728, 2006.
Xu et al., Circ. Res., 91(6):501-8, 2002.
Xu et al., Nat. Biotechnol., 19:971-974, 2001.
Yang and Russell, Proc. Natl. Acad. Sci. USA, 87:4144-4148,1990.
Ying et al., Cell, 115:281-292,2003.
Yoo et al., J. Bone Joint Sure. Am., 80(12):1745-1757, 1998.
Yu and Thompson, Genes Dev., 22(15):1987-97, 2008.
Yu et al., Science, 318:1917-1920, 2007.
Zufferey et al., Nat. Biotechnol., 15(9):871-875, 1997.

## Claims

1. A method of testing a compound for its effect on differentiation of specific cells or tissue types, comprising the steps of:
(a) providing an *in vitro* set of cell lines comprising at least a first and second pluripotent stem cell line, wherein said first and second lines respectively comprise an integrated exogenous expression cassette, wherein the exogenous expression cassettes from the first and second lines comprise a selectable or screenable marker under the control of a differentiation-responsive regulatory element, wherein the differentiation-responsive regulatory element of said exogenous expression cassette of the first cell line is different from the differentiation-responsive regulatory element of the exogenous expression cassette of the second cell line, such that the marker of the cassette in the first cell line is expressed only if the cell is in a first differentiation state and the marker of the cassette in the second cell line is expressed only if the cell is in a second differentiation state and wherein the expression of the selectable or screenable markers from the exogenous expression cassettes of the first and second cell lines can be screened or selected using the same method,
(b) culturing the pluripotent stem cell lines under a differentiation condition in the presence of a test compound;
(c) determining the expression of the selectable or screenable marker for the effect of the testing compound on the differentiation of the pluripotent stem cell to the selected cell lineages or tissue types;
wherein the pluripotent stem cells comprise one or more human induced pluripotent stem (iPS) cells.

2. The method of claim 1, wherein the iPS cells are essentially free of exogenous retroviral genetic elements.

3. The method of claim 1, wherein the exogenous expression cassette of the first or second cell line is comprised at a predetermined location of the genome of the pluripotent stem cells.

4. The method of claim 3, wherein one or more cell lines of the set comprise an additional exogenous expression cassette comprised in a transposon system.

5. The method of claim 4, wherein the additional exogenous expression cassette in each cell line includes a selectable or screenable marker under the control of a drug-responsive regulatory element.

6. The method of claim 1, comprising at least five to ten different pluripotent stem cell lines, each comprising a different exogenous expression cassette having a different differentiation-responsive regulatory element.

7. The method of claim 1, wherein the exogenous expression cassette of the first or second cell line comprises at least two separate exogenous expression cassettes, each comprising a different condition-responsive regulatory element.

8. The method of claim 1, wherein each pluripotent stem cell line is contained in a separate container different from other cell lines in the set of cell lines.

9. The method of claim 1, wherein the differentiation-responsive regulatory element comprises a tissue-specific promoter, or a cell-specific promoter which causes expression of a selectable or screenable marker when the pluripotent stem cell of the cell line differentiates to a selected cell lineage.

10. The method of claim 1, wherein one or more cell lines of the set of cell lines comprise an additional exogenous expression cassette including a selectable or screenable marker under the control of a drug-responsive regulatory element,

11. The method of claim 10, wherein the additional exogenous expression cassette is comprised in a transposon system.

12. The method of claim 9, wherein the cell-specific promoter is a neural progenitor-specific promoter, a hepatocyte progenitor-specific promoter, a hematopoietic progenitor-specific promoter or a cardiac progenitor-specific promoter,is a promoter specific for a selected terminally differentiated cell, or is a ventricular cardiomyocyte-specific promoter, an atrial cardiomyocyte-specific promoter, a nodal cardiomyocyte-specific promoter, an arterial endothelial cell-specific promoter, a venous endothelial cell-specific promoter, a lymphatic endothelial cell-specific promoter, a blood-brain barrier endothelial cell-specific promoter, a dopaminergic neuron-specific promoter, a cholinergic neuron-specific promoter, a gabaergic neuron-specific promoter, or a motor neuron-specific promoter.

13. The method of claim 9, wherein the tissue-specific promoter comprises a kidney-specific promoter, a kidney medulla-specific promoter, a kidney cortex-specific promoter, a heart-specific promoter, a pan-cardiac promoter, a heart atria-specific promoter, a heart ventricle-specific promoter, a liver-specific promoter, a neural-specific promoter, a pancreas-specific promoter, a lung-specific promoter, an endothelial-specific promoter, a blood-specific promoter or an intestine-specific promoter.

14. The method of claim 5, wherein the drug-responsive regulatory element comprises a promoter of a drug metabolizing enzyme gene.

15. The method of claim 14, wherein the promoter is a promoter of a gene encoding a cytochrome P450 monooxygenase, N-acetyltransferase, thiopurine methyltransferase or dihydropyrimidine dehydrogenase.

16. The method of claim 5, wherein the drug-responsive regulatory element comprises a drug signaling pathway-responsive promoter which causes expression of a selectable or screenable marker in a cell where the drug-responsive signaling pathway is activated.

17. The method of claim 16, wherein the drug signaling pathway is a tyrosine kinase pathway, heterotrimeric G protein pathway, small GTPase pathway, serine/threonine protein kinase pathway, phosphatase pathway, lipid kinase pathway, hydrolase pathway, cyclic AMP (cAMP)-mediated pathway, cyclic GMP (cGMP)-mediated pathway, phosphatidylinositol-triphosphate (PIP3)-mediated pathway, diacylglycerol (DAG)-mediated pathway, inositol-triphosphate (IP3)-mediated pathway, EF hand domains of calmodulin-mediated signaling pathway, pleckstrin homology domains of the kinase protein AKT-mediated signaling pathway, chromatin regulation signaling pathway, MAPK signaling pathway, apoptosis/autophagy pathway, translational control pathway, cell cycle/checkpoint pathway, DNA damage pathway, Jak/Stat signaling pathway, NF-κB signaling pathway, TGF-β/Smad signaling pathway, lymphocyte signaling pathway, angiogenesis pathway, vesicle trafficking pathway, cytoskeletal signaling pathway, adhesion pathway, glucose metabolism pathway, Wnt/Hedgehog/Notch signaling pathway, stem cell lineage specification pathway, nuclear receptor-mediated pathway, or protein folding and stability signaling pathway.

18. The method of claim 1, wherein the selectable marker comprises an antibiotic resistance gene or an antigenic epitope or wherein the screenable marker is further defined as a gene that expresses a fluorescent, luminescent or bioluminescent protein.

19. The method of claim 1, wherein the method of testing a compound for its effect on differentiation of specific cells or tissue types is a method for testing a drug candidate for its efficacy and/or toxicity.

## Patentansprüche

1. Verfahren zum Testen einer Verbindung auf deren Wirkung auf die Differenzierung von spezifischen Zellen oder Gewebearten, umfassend die Schritte:
(a) Bereitstellen eines *in vitro*-Zellliniensets, das mindestens eine erste und eine zweite pluripotente Stammzelllinie umfasst, wobei die erste und zweite Zelllinie jeweils eine integrierte exogene Expressionskassette umfassen, wobei die exogenen Expressionskassetten der ersten und zweiten Linie einen selektierbaren oder screenbaren Marker unter der Kontrolle eines auf eine Differenzierung reagierenden Regulationselements (differentiation-responsive regulatory element) umfassen, wobei das auf eine Differenzierung reagierende Regulationselement der exogenen Expressionskassette der ersten Zelllinie ein anderes ist als das auf eine Differenzierung reagierende Regulationselement der exogenen Expressionskassette der zweiten Zelllinie, so dass der Marker der Kassette in der ersten Zelllinie nur exprimiert wird, wenn die Zelle in einem ersten Differenzierungsstadium ist und der Marker der Kassette in der zweiten Zelllinie nur exprimiert wird, wenn die Zelle in einem zweiten Differenzierungsstadium ist und wobei die Expression der selektierbaren oder screenbaren Marker der exogenen Expressionskassetten der ersten und zweiten Zelllinie unter Verwendung des gleichen Verfahrens gescreent oder selektiert werden kann,
(b) Züchten der pluripotenten Stammzelllinien unter Differenzierungsbedingungen in Anwesenheit einer Testverbindung;
(c) Bestimmen der Expression des selektierbaren oder screenbaren Markers auf die Wirkung der Testverbindung auf die Differenzierung der pluripotenten Stammzelle zu den selektierten Zellabstammungen oder Gewebearten;
wobei die pluripotenten Stammzellen eine oder mehrere humane induzierte pluripotente Stamm (iPS)-Zellen umfassen.

2. Verfahren nach Anspruch 1, wobei die iPS-Zellen im Wesentlichen frei von exogenen retroviralen genetischen Elementen sind.

3. Verfahren nach Anspruch 1, wobei die exogene Expressionskassette der ersten oder zweiten Zelllinie an einer vorher bestimmten Position des Genoms der pluripotenten Stammzellen umfasst ist.

4. Verfahren nach Anspruch 3, wobei die ein oder mehreren Zelllinien des Sets eine zusätzliche exogene Expressionskassette umfassen, die in einem Transposon-System umfasst ist.

5. Verfahren nach Anspruch 4, wobei die zusätzliche exogene Expressionskassette in jeder Zelllinie einen selektierbaren oder screenbaren Marker unter der Kontrolle eines auf einen Wirkstoff reagierenden Regulationselements (drug-responsive regulatory element) einschließt.

6. Verfahren nach Anspruch 1, das mindestens fünf bis zehn verschiedene pluripotente Stammzelllinien umfasst, wobei jede eine unterschiedliche exogene Expressionskassette umfasst, die ein auf eine unterschiedliche Differenzierung reagierendes Regulationselement hat.

7. Verfahren nach Anspruch 1, wobei die exogene Expressionskassette der ersten oder zweiten Zelllinie mindestens zwei separate exogene Expressionskassetten umfasst, wobei jede ein auf einen anderen Zustand reagierendes Regulationselement umfasst.

8. Verfahren nach Anspruch 1, wobei jede pluripotente Stammzelllinie in einem separaten Behälter enthalten ist, der sich von den anderen Zelllinien im Set der Zelllinien unterscheidet.

9. Verfahren nach Anspruch 1, wobei das auf eine Differenzierung reagierende Regulationselement einen Gewebe-spezifischen Promotor oder einen Zellspezifischen Promotor umfasst, der die Expression eines selektierbaren oder screenbaren Markers verursacht, wenn die pluripotente Stammzelle der Zelllinie zu einer selektierten Zellabstammung differenziert.

10. Verfahren nach Anspruch 1, wobei die ein oder mehreren Zelllinien des Zelllinien-Sets eine zusätzliche exogene Expressionskassette umfassen, die einen selektierbaren oder screenbaren Marker unter der Kontrolle eines auf einen Wirkstoff reagierenden Regulationselements einschließen.

11. Verfahren nach Anspruch 10, wobei die zusätzliche exogene Expressionskassette in einem Transposon-System umfasst ist.

12. Verfahren nach Anspruch 9, wobei der Zell-spezifische Promotor ein neuronaler Progenitor-spezifischer Promotor, ein hepatocyter Progenitor-spezifischer Promotor, ein hematopoietischer Progenitor-spezifischer Promotor oder ein kardialer Progenitor-spezifischer Promotor, ein Promotor, der spezifisch für eine ausgewählte terminal-differenzierte Zelle ist oder ein ventrikulärer Kardiomyozyten-spezifischer Promotor, ein atrialer Kardiomyozyten-spezifischer Promotor, ein nodaler Kardiomyozyten-spezifischer Promotor, ein arterieller Endothelzell-spezifischer Promotor, ein venöser Endothelzell-spezifischer Promotor, ein lymphatischer Endothelzell-spezifischer Promotor, ein Blut-Hirn-Schranke-Endothelzell-spezifischer Promotor, ein dopaminerger Neuronen-spezifischer Promotor, ein cholinerger Neuronen-spezifischer Promotor, ein GABAerger Neuronen-spezifischer Promotor oder ein Motorneuronen-spezifischer Promotor ist.

13. Verfahren nach Anspruch 9, wobei der Gewebe-spezifische Promotor einen Nieren-spezifischen Promotor, einen Nierenmark-spezifischen Promotor, einen Nierenrinde-spezifischen Promotor, einen Herz-spezifischen Promotor, einen Pan-Herz-spezifischen Promotor, einen Herz-Vorhof-spezifischen Promotor, einen Herzventrikel-spezifischen Promotor, einen Leber-spezifischen Promotor, einen Neuronen-spezifischen Promotor, einen Pankreas-spezifischen Promotor, einen Lungen-spezifischen Promotor, einen Endothel-spezifischen Promotor, einen Blut-spezifischen Promotor oder einen Darm-spezifischen Promotor umfasst.

14. Verfahren nach Anspruch 5, wobei das auf einen Wirkstoff reagierende Regulationselement einen Promotor eines Wirkstoff-metabolisierenden Enzymgens umfasst.

15. Verfahren nach Anspruch 14, wobei der Promotor ein Promotor eines Gens ist, das eine cytochrome P450-Monooxygenase, N-Acetyltransferase, Thiopurinmethyltransferase oder Dihydropyrimidin-Dehydrogenase codiert.

16. Verfahren nach Anspruch 5, wobei das auf einen Wirkstoff reagierende Regulationselement einen Promotor umfasst, der auf einen Wirkstoff-Signalweg reagiert, der die Expression eines selektierbaren oder screenbaren Markers in einer Zelle verursacht, in der der auf einen Wirkstoff reagierende Signalweg aktiviert ist.

17. Verfahren nach Anspruch 16, wobei der Wirkstoff-Signalweg ein Tyrosinkinase-Signalweg, ein heterotrimeres-G-Protein-Signalweg, ein kleine-GTPase-Signalweg, ein SerinfThreonin-Protein-Kinase-Signalweg, ein Phosphatase-Signalweg, ein Lipid-Kinase-Signalweg, ein Hydrolase-Signalweg, ein cyklische AMP (cAMP)-vermittelter Signalweg, ein cyklische GMP (cGMP)-vermittelter Signalweg, Phosphatidylinositol-Triphosphat (PIP3)-vermittelter Signalweg, Diacylglycerol (DAG)-vermittelter Signalweg, Inositol-triphosphat (IP3)-vermittelter Signalweg, EF-Handdomänen des Calmodulin-vermittelten Signalwegs, Pleckstrin-Homologie-Domänen des Kinaseprotein AKTvermittelten Signalwegs, Chromatinregulation-Signalweg, MAPK-Signalweg, Apoptose/Autophagie-Signalweg, Translationskontrolle-Signalweg, Zellzyklus/Checkpoint-Signalweg, DNA-Schaden-Signalweg, Jak/Stat-Signalweg, NF-κB-Signalweg, TGF-β/Smad-Signalweg, Lymphozyten-Signalweg, Angiogenese-Signalweg, Vesikel-transportierender Signalweg, Cytoskelett-Signalweg, Adhäsion-Signalweg, Glucose-Metabolismus-Signalweg, Wnt/Hedgehog/Notch-Signalweg, Stammzelllinie-Spezifikation-Signalweg, nukleärer Rezeptor-vermittelter Signalweg oder Proteinfaltung und -stabilität-Signalweg ist.

18. Verfahren nach Anspruch 1, wobei der selektierbare Marker ein Antibiotikaresistenzgen oder ein antigenes Epitop umfasst, oder wobei der screenbare Marker des Weiteren als Gen definiert ist, das ein fluoreszierendes, lumineszierendes oder biolumineszierendes Protein exprimiert.

19. Verfahren nach Anspruch 1, wobei das Verfahren zum Testen einer Verbindung auf seine Wirkung auf die Differenzierung spezifischer Zellen oder Gewebetypen ein Verfahren zum Testen eines Wirkstoffkandidaten auf seine Wirksamkeit und/oder Toxizität ist.

## Revendications

1. Procédé de test d'un composé pour son effet sur la différenciation de cellules spécifiques ou de types de tissus spécifiques, comprenant les étapes de:
(a) fourniture d'un ensemble *in vitro* de lignées cellulaires comprenant au moins une première et une seconde lignée de cellules souches pluripotentes, où lesdites première et seconde lignées comprennent respectivement une cassette d'expression exogène intégrée, où les cassettes d'expression exogènes provenant des première et seconde lignées comprennent un marqueur sélectionnable ou criblable sous le contrôle d'un élément régulateur sensible à la différenciation, où l'élément régulateur sensible à la différenciation de ladite cassette d'expression exogène de la première lignée cellulaire est différent de l'élément régulateur sensible à la différenciation de la cassette d'expression exogène de la seconde lignée cellulaire, de sorte que le marqueur de la cassette dans la première lignée cellulaire est exprimé seulement si la cellule est dans un premier état de différenciation et le marqueur de la cassette dans la seconde lignée cellulaire est exprimé seulement si la cellule est dans un second état de différenciation et où l'expression des marqueurs sélectionnables ou criblables provenant des cassettes d'expression exogènes des première et seconde lignées cellulaires peut être criblée ou sélectionnée au moyen du même procédé,
(b) culture des lignées de cellules souches pluripotentes dans des conditions de différenciation en présence d'un composé test;
(c) détermination de l'expression du marqueur sélectionnable ou criblable pour l'effet du composé testé sur la différenciation de la cellule souche pluripotente en les lignées cellulaires ou les types de tissus sélectionnés;
où les cellules souches pluripotentes comprennent une ou plusieurs cellules souches pluripotentes induites (CSPi) humaines.

2. Procédé selon la revendication 1, où les cellules CSPi sont sensiblement exemptes d'éléments génétiques rétroviraux exogènes.

3. Procédé selon la revendication 1, où la cassette d'expression exogène de la première ou seconde lignée cellulaire est comprise à un emplacement prédéterminé du génome des cellules souches pluripotentes.

4. Procédé selon la revendication 3, où une ou plusieurs lignées cellulaires de l'ensemble comprennent une cassette d'expression exogène supplémentaire comprise dans un système de transposon.

5. Procédé selon la revendication 4, où la cassette d'expression exogène supplémentaire dans chaque lignée cellulaire inclut un marqueur sélectionnable ou criblable sous le contrôle d'un élément régulateur sensible à un médicament.

6. Procédé selon la revendication 1, comprenant au moins cinq à dix lignées de cellules souches pluripotentes différentes, comprenant chacune une cassette d'expression exogène différente ayant un élément régulateur sensible à la différenciation différent.

7. Procédé selon la revendication 1, où la cassette d'expression exogène de la première ou seconde lignée cellulaire comprend au moins deux cassettes d'expression exogènes séparées, comprenant chacune un élément régulateur sensible aux conditions différent.

8. Procédé selon la revendication 1, où chaque lignée de cellules souches pluripotentes est contenue dans un récipient séparé différent d'autres lignées cellulaires dans l'ensemble de lignées cellulaires.

9. Procédé selon la revendication 1, où l'élément régulateur sensible à la différenciation comprend un promoteur spécifique de tissu, ou un promoteur spécifique de cellule qui provoque l'expression d'un marqueur sélectionnable ou criblable quand la cellule souche pluripotente de la lignée cellulaire se différencie en une lignée cellulaire sélectionnée.

10. Procédé selon la revendication 1, où une ou plusieurs lignées cellulaires de l'ensemble de lignées cellulaires comprennent une cassette d'expression exogène supplémentaire incluant un marqueur sélectionnable ou criblable sous le contrôle d'un élément régulateur sensible à un médicament.

11. Procédé selon la revendication 10, où la cassette d'expression exogène supplémentaire est comprise dans un système de transposon.

12. Procédé selon la revendication 9, où le promoteur spécifique de cellule est un promoteur spécifique de progéniteur neural, un promoteur spécifique de progéniteur hépatocyte, un promoteur spécifique de progéniteur hématopoïétique ou un promoteur spécifique de progéniteur cardiaque, est un promoteur spécifique pour une cellule différenciée de manière terminale sélectionnée, ou est un promoteur spécifique de cardiomyocyte ventriculaire, un promoteur spécifique de cardiomyocyte auriculaire, un promoteur spécifique de cardiomyocyte nodal, un promoteur spécifique de cellule endothéliale artérielle, un promoteur spécifique de cellule endothéliale veineuse, un promoteur spécifique de cellule endothéliale lymphatique, un promoteur spécifique de cellule endothéliale de la barrière hémato-encéphalique, un promoteur spécifique de neurone dopaminergique, un promoteur spécifique de neurones cholinergique, un promoteur spécifique de neurone gabaergique ou un promoteur spécifique de neurone moteur.

13. Procédé selon la revendication 9, où le promoteur spécifique de tissu comprend un promoteur spécifique du rein, un promoteur spécifique de la médulla du rein, un promoteur spécifique du cortex du rein, un promoteur spécifique du coeur, un promoteur pan-cardiaque, un promoteur spécifique des oreillettes du coeur, un promoteur spécifique des ventricules du coeur, un promoteur spécifique du foie, un promoteur spécifique du système nerveux, un promoteur spécifique du pancréas, un promoteur spécifique du poumon, un promoteur spécifique de l'endothélium, un promoteur spécifique du sang ou un promoteur spécifique de l'intestin.

14. Procédé selon la revendication 5, où l'élément régulateur sensible à un médicament comprend un promoteur d'un gène d'enzyme métabolisant un médicament.

15. Procédé selon la revendication 14, où le promoteur est un promoteur d'un gène codant une cytochrome P450 monooxygénase, une N-acétyltransférase, une thiopurine méthyltransférase ou une dihydropyrimidine déshydrogénase.

16. Procédé selon la revendication 5, où l'élément régulateur sensible à un médicament comprend un promoteur sensible à une voie de signalisation de médicament qui provoque l'expression d'un marqueur sélectionnable ou criblable dans une cellule où la voie de signalisation sensible à un médicament est activée.

17. Procédé selon la revendication 16, où la voie de signalisation de médicament est une voie de tyrosine kinase, une voie de protéine G hétérotrimérique, une voie de petite GTPase, une voie de sérine/thréonine protéine kinase, une voie de phosphatase, une voie de lipide kinase, une voie d'hydrolase, une voie médiée par l'AMP cyclique (AMPc), une voie médiée par le GMP cyclique (GMPc), une voie médiée par le phosphatidylinositol-triphosphate (PIP3), une voie médiée par le diacylglycérol (DAG), une voie médiée par l'inositol-triphosphate (IP3), une voie de signalisation médiée par les domaines de main EF de la calmoduline, une voie de signalisation médiée par les domaines homologues de la pleckstrine de la protéine kinase AKT, une voie de signalisation de régulation de la chromatine, une voie de signalisation de MAPK, une voie d'apoptose/autophagie, une voie de contrôle traductionnel, une voie de cycle/point de contrôle cellulaire, une voie de détérioration d'ADN, une voie de signalisation Jak/Stat, une voie de signalisation de NF-κB, une voie de signalisation de TGF-β/Smad, une voie de signalisation des lymphocytes, une voie d'angiogenèse, une voie de transport de vésicules, une voie de signalisation du cytosquelette, une voie d'adhésion, une voie du métabolisme du glucose, une voie de signalisation de Wnt/Hedgehog/Notch, une voie de spécification de lignée de cellules souches, une voie médiée par un récepteur nucléaire ou une voie de signalisation du repliement et de la stabilité des protéines.

18. Procédé selon la revendication 1, où le marqueur sélectionnable comprend un gène de résistance à un antibiotique ou un épitope antigénique ou bien où le marqueur criblable est défini en outre comme un gène qui exprime une protéine fluorescente, luminescente ou bioluminescente.

19. Procédé selon la revendication 1, où le procédé de test d'un composé pour son effet sur la différenciation de cellules spécifiques ou de types de tissus spécifiques est un procédé pour tester un candidat médicament pour son efficacité et/ou sa toxicité.
